# EUROPÄISCHE PATENTANMELDUNG

(11) **EP 3 722 437 A1**
(43) Veröffentlichungstag der Anmeldung: **14.10.2020**
(21) Anmeldenummer: 20169379.3
(22) Anmeldetag: 15.04.2015
(51) Int. Cl.: C12N 15/861, C07K 14/005

(54) **VIRALER VEKTOR FÜR DEN ZIELGERICHTETEN GENTRANSFER IN GEHIRN UND RÜCKENMARK**

(30) Priorität: 17.04.2014 DE 102014207498
(62) Teilanmeldung aus: 19153559.0
(71) Anmelder: Boehringer Ingelheim International GmbH, 55216 Ingelheim am Rhein (DE)
(72) Erfinder: KOERBELIN, Jakob, 22769 Hamburg (DE); MICHELFELDER, Stefan, 79211 Denzlingen (DE); TREPEL, Martin, 22149 Hamburg (DE)
(74) Vertreter: Uexküll & Stolberg

(57) **Zusammenfassung**

Die Erfindung betrifft neuartige Peptide, Polypeptide oder Proteine, die spezifisch an Zellen des Gehirns und/oder des Rückenmarks binden. Die Peptide, Polypeptide oder Proteine können Bestandteil eines viralen Kapsids sein und dazu verwendet werden, einen rekombinanten viralen Vektor nach systemischer Verabreichung an ein Subjekt selektiv zum Gehirn und/oder zum Rückenmark zu leiten und dort für eine gewebespezifische Expression von einem oder mehreren Transgenen zu sorgen. Die Erfindung betrifft somit auch einen rekombinanten viralen Vektor, vorzugsweise einen AAV-Vektor, der ein Kapsid umfasst, welches mindestens eines der erfindungsgemäßen Peptide, Polypeptide oder Proteine enthält, sowie mindestens ein in dem Kapsid verpacktes Transgen. Der virale Vektor eignet sich insbesondere zur therapeutischen Behandlung einer Erkrankung oder Funktionsstörung des Gehirns und/oder des Rückenmarks. Die Erfindung betrifft ferner Zellen und pharmazeutische Zusammensetzungen, die den erfindungsgemäßen viralen Vektor umfassen.

SEQUENCE LISTING

<110>  Boehringer Ingelheim International GmbH

<120>  Neue Peptide mit Spezifität für Gehirn und Rückenmark

<130>  P 109319

<160>  42

<170>  PatentIn version 3.5

<210>  1
<211>  7
<212>  PRT
<213>  Künstliche Sequenz

<220>
<223>  Gehirnspezifisches Peptid

<400>  1

Asn Arg Gly Thr Glu Trp Asp
1               5


<210>  2
<211>  7
<212>  PRT
<213>  Künstliche Sequenz

<220>
<223>  Gehirnspezifisches Peptid

<400>  2

Ala Asp Gly Val Gln Trp Thr
1               5


<210>  3
<211>  7
<212>  PRT
<213>  Künstliche Sequenz

<220>
<223>  Gehirnspezifisches Peptid

<400>  3

Asp Asp Gly Val Ser Trp Lys
1               5


<210>  4
<211>  7
<212>  PRT
<213>  Künstliche Sequenz

<220>
<223>  Gehirnspezifisches Peptid

<400>  4

Ser Asp Gly Leu Thr Trp Ser
1               5

<210>  5
<211>  7
<212>  PRT
<213>  Künstliche Sequenz

<220>
<223>  Gehirnspezifisches Peptid

<400>  5

Ser Asp Gly Leu Ala Trp Val
1               5

<210>  6
<211>  7
<212>  PRT
<213>  Künstliche Sequenz

<220>
<223>  Konsensus-Sequenz

<220>
<221>  misc_feature
<222>  (1)..(1)
<223>  Xaa kann jede natürlich vorkommende Aminosäure sein

<220>
<221>  misc_feature
<222>  (4)..(5)
<223>  Xaa kann jede natürlich vorkommende Aminosäure sein

<220>
<221>  misc_feature
<222>  (7)..(7)
<223>  Xaa kann jede natürlich vorkommende Aminosäure sein

<400>  6

Xaa Asp Gly Xaa Xaa Trp Xaa
1               5

<210>  7
<211>  735
<212>  PRT
<213>  Adeno-assoziierte Virus 2

<400>  7

<210>  8
<211>  598
<212>  PRT
<213>  Adeno-assoziierte Virus 2

<400>  8

Met Ala Pro Gly Lys Lys Arg Pro Val Glu His Ser Pro Val Glu Pro
1               5                   10                  15

Asp Ser Ser Ser Gly Thr Gly Lys Ala Gly Gln Gln Pro Ala Arg Lys
            20                  25                  30

Arg Leu Asn Phe Gly Gln Thr Gly Asp Ala Asp Ser Val Pro Asp Pro
        35                  40                  45

Gln Pro Leu Gly Gln Pro Pro Ala Ala Pro Ser Gly Leu Gly Thr Asn
    50                  55                  60

Thr Met Ala Thr Gly Ser Gly Ala Pro Met Ala Asp Asn Asn Glu Gly
65                  70                  75                  80

Ala Asp Gly Val Gly Asn Ser Ser Gly Asn Trp His Cys Asp Ser Thr
                85                  90                  95

Trp Met Gly Asp Arg Val Ile Thr Thr Ser Thr Arg Thr Trp Ala Leu
            100                 105                 110

Pro Thr Tyr Asn Asn His Leu Tyr Lys Gln Ile Ser Ser Gln Ser Gly
        115                 120                 125

Ala Ser Asn Asp Asn His Tyr Phe Gly Tyr Ser Thr Pro Trp Gly Tyr
    130                 135                 140

Phe Asp Phe Asn Arg Phe His Cys His Phe Ser Pro Arg Asp Trp Gln
145                 150                 155                 160

Arg Leu Ile Asn Asn Asn Trp Gly Phe Arg Pro Lys Arg Leu Asn Phe
                165                 170                 175

Lys Leu Phe Asn Ile Gln Val Lys Glu Val Thr Gln Asn Asp Gly Thr
            180                 185                 190

Thr Thr Ile Ala Asn Asn Leu Thr Ser Thr Val Gln Val Phe Thr Asp
        195                 200                 205

Ser Glu Tyr Gln Leu Pro Tyr Val Leu Gly Ser Ala His Gln Gly Cys
    210                 215                 220

Leu Pro Pro Phe Pro Ala Asp Val Phe Met Val Pro Gln Tyr Gly Tyr
225                 230                 235                 240

Leu Thr Leu Asn Asn Gly Ser Gln Ala Val Gly Arg Ser Ser Phe Tyr
                245                 250                 255

Cys Leu Glu Tyr Phe Pro Ser Gln Met Leu Arg Thr Gly Asn Asn Phe
            260                 265                 270

Thr Phe Ser Tyr Thr Phe Glu Asp Val Pro Phe His Ser Ser Tyr Ala

275                 280                 285

His Ser Gln Ser Leu Asp Arg Leu Met Asn Pro Leu Ile Asp Gln Tyr
    290                 295                 300

Leu Tyr Tyr Leu Ser Arg Thr Asn Thr Pro Ser Gly Thr Thr Thr Gln
305                 310                 315                 320

Ser Arg Leu Gln Phe Ser Gln Ala Gly Ala Ser Asp Ile Arg Asp Gln
                325                 330                 335

Ser Arg Asn Trp Leu Pro Gly Pro Cys Tyr Arg Gln Gln Arg Val Ser
            340                 345                 350

Lys Thr Ser Ala Asp Asn Asn Asn Ser Glu Tyr Ser Trp Thr Gly Ala
        355                 360                 365

Thr Lys Tyr His Leu Asn Gly Arg Asp Ser Leu Val Asn Pro Gly Pro
    370                 375                 380

Ala Met Ala Ser His Lys Asp Asp Glu Glu Lys Phe Phe Pro Gln Ser
385                 390                 395                 400

Gly Val Leu Ile Phe Gly Lys Gln Gly Ser Glu Lys Thr Asn Val Asp
                405                 410                 415

Ile Glu Lys Val Met Ile Thr Asp Glu Glu Glu Ile Arg Thr Thr Asn
            420                 425                 430

Pro Val Ala Thr Glu Gln Tyr Gly Ser Val Ser Thr Asn Leu Gln Arg
        435                 440                 445

Gly Asn Arg Gln Ala Ala Thr Ala Asp Val Asn Thr Gln Gly Val Leu
    450                 455                 460

Pro Gly Met Val Trp Gln Asp Arg Asp Val Tyr Leu Gln Gly Pro Ile
465                 470                 475                 480

Trp Ala Lys Ile Pro His Thr Asp Gly His Phe His Pro Ser Pro Leu
                485                 490                 495

Met Gly Gly Phe Gly Leu Lys His Pro Pro Pro Gln Ile Leu Ile Lys
            500                 505                 510

Asn Thr Pro Val Pro Ala Asn Pro Ser Thr Thr Phe Ser Ala Ala Lys
        515                 520                 525

Phe Ala Ser Phe Ile Thr Gln Tyr Ser Thr Gly Gln Val Ser Val Glu
    530                 535                 540

Ile Glu Trp Glu Leu Gln Lys Glu Asn Ser Lys Arg Trp Asn Pro Glu
545                 550                 555                 560

Ile Gln Tyr Thr Ser Asn Tyr Asn Lys Ser Val Asn Val Asp Phe Thr
                565                 570                 575

Val Asp Thr Asn Gly Val Tyr Ser Glu Pro Arg Pro Ile Gly Thr Arg
            580                 585                 590

Tyr Leu Thr Arg Asn Leu
        595

<210>  9
<211>  533
<212>  PRT
<213>  Adeno-assoziierte Virus 2

<400>  9

Met Ala Thr Gly Ser Gly Ala Pro Met Ala Asp Asn Asn Glu Gly Ala
1               5                   10                  15

Asp Gly Val Gly Asn Ser Ser Gly Asn Trp His Cys Asp Ser Thr Trp
            20                  25                  30

Met Gly Asp Arg Val Ile Thr Thr Ser Thr Arg Thr Trp Ala Leu Pro
        35                  40                  45

Thr Tyr Asn Asn His Leu Tyr Lys Gln Ile Ser Ser Gln Ser Gly Ala
    50                  55                  60

Ser Asn Asp Asn His Tyr Phe Gly Tyr Ser Thr Pro Trp Gly Tyr Phe
65                  70                  75                  80

Asp Phe Asn Arg Phe His Cys His Phe Ser Pro Arg Asp Trp Gln Arg
                85                  90                  95

Leu Ile Asn Asn Asn Trp Gly Phe Arg Pro Lys Arg Leu Asn Phe Lys
            100                 105                 110

Leu Phe Asn Ile Gln Val Lys Glu Val Thr Gln Asn Asp Gly Thr Thr
        115                 120                 125

Thr Ile Ala Asn Asn Leu Thr Ser Thr Val Gln Val Phe Thr Asp Ser
    130                 135                 140

Glu Tyr Gln Leu Pro Tyr Val Leu Gly Ser Ala His Gln Gly Cys Leu
145                 150                 155                 160

Pro Pro Phe Pro Ala Asp Val Phe Met Val Pro Gln Tyr Gly Tyr Leu
                165                 170                 175

Thr Leu Asn Asn Gly Ser Gln Ala Val Gly Arg Ser Ser Phe Tyr Cys
            180                 185                 190

Leu Glu Tyr Phe Pro Ser Gln Met Leu Arg Thr Gly Asn Asn Phe Thr
        195                 200                 205

Phe Ser Tyr Thr Phe Glu Asp Val Pro Phe His Ser Ser Tyr Ala His
    210                 215                 220

Ser Gln Ser Leu Asp Arg Leu Met Asn Pro Leu Ile Asp Gln Tyr Leu
225                 230                 235                 240

Tyr Tyr Leu Ser Arg Thr Asn Thr Pro Ser Gly Thr Thr Thr Gln Ser
                245                 250                 255

Arg Leu Gln Phe Ser Gln Ala Gly Ala Ser Asp Ile Arg Asp Gln Ser
            260                 265                 270

Arg Asn Trp Leu Pro Gly Pro Cys Tyr Arg Gln Gln Arg Val Ser Lys
        275                 280                 285

Thr Ser Ala Asp Asn Asn Asn Ser Glu Tyr Ser Trp Thr Gly Ala Thr
    290                 295                 300

Lys Tyr His Leu Asn Gly Arg Asp Ser Leu Val Asn Pro Gly Pro Ala
305                 310                 315                 320

Met Ala Ser His Lys Asp Asp Glu Glu Lys Phe Phe Pro Gln Ser Gly
                325                 330                 335

Val Leu Ile Phe Gly Lys Gln Gly Ser Glu Lys Thr Asn Val Asp Ile
            340                 345                 350

Glu Lys Val Met Ile Thr Asp Glu Glu Glu Ile Arg Thr Thr Asn Pro
        355                 360                 365

Val Ala Thr Glu Gln Tyr Gly Ser Val Ser Thr Asn Leu Gln Arg Gly
    370                 375                 380

Asn Arg Gln Ala Ala Thr Ala Asp Val Asn Thr Gln Gly Val Leu Pro
385                 390                 395                 400

Gly Met Val Trp Gln Asp Arg Asp Val Tyr Leu Gln Gly Pro Ile Trp
                405                 410                 415

Ala Lys Ile Pro His Thr Asp Gly His Phe His Pro Ser Pro Leu Met
            420                 425                 430

Gly Gly Phe Gly Leu Lys His Pro Pro Pro Gln Ile Leu Ile Lys Asn
        435                 440                 445

Thr Pro Val Pro Ala Asn Pro Ser Thr Thr Phe Ser Ala Ala Lys Phe
    450                 455                 460

Ala Ser Phe Ile Thr Gln Tyr Ser Thr Gly Gln Val Ser Val Glu Ile
465                 470                 475                 480

Glu Trp Glu Leu Gln Lys Glu Asn Ser Lys Arg Trp Asn Pro Glu Ile
                485                 490                 495

Gln Tyr Thr Ser Asn Tyr Asn Lys Ser Val Asn Val Asp Phe Thr Val
            500                 505                 510

Asp Thr Asn Gly Val Tyr Ser Glu Pro Arg Pro Ile Gly Thr Arg Tyr
        515                 520                 525

Leu Thr Arg Asn Leu
    530

<210> 10
<211> 744
<212> PRT
<213> Adeno-assoziierte Virus 2

<400> 10

Met Ala Ala Asp Gly Tyr Leu Pro Asp Trp Leu Glu Asp Thr Leu Ser
1               5                   10                  15

Glu Gly Ile Arg Gln Trp Trp Lys Leu Lys Pro Gly Pro Pro Pro Pro
            20                  25                  30

Lys Pro Ala Glu Arg His Lys Asp Asp Ser Arg Gly Leu Val Leu Pro
        35                  40                  45

Gly Tyr Lys Tyr Leu Gly Pro Phe Asn Gly Leu Asp Lys Gly Glu Pro
    50                  55                  60

Val Asn Glu Ala Asp Ala Ala Ala Leu Glu His Asp Lys Ala Tyr Asp
65                  70                  75                  80

Arg Gln Leu Asp Ser Gly Asp Asn Pro Tyr Leu Lys Tyr Asn His Ala
                85                  90                  95

Asp Ala Glu Phe Gln Glu Arg Leu Lys Glu Asp Thr Ser Phe Gly Gly
            100                 105                 110

Asn Leu Gly Arg Ala Val Phe Gln Ala Lys Lys Arg Val Leu Glu Pro
        115                 120                 125

Leu Gly Leu Val Glu Glu Pro Val Lys Thr Ala Pro Gly Lys Lys Arg
    130                 135                 140

Pro Val Glu His Ser Pro Val Glu Pro Asp Ser Ser Ser Gly Thr Gly
145                 150                 155                 160

Lys Ala Gly Gln Gln Pro Ala Arg Lys Arg Leu Asn Phe Gly Gln Thr
                165                 170                 175

Gly Asp Ala Asp Ser Val Pro Asp Pro Gln Pro Leu Gly Gln Pro Pro
            180                 185                 190

Ala Ala Pro Ser Gly Leu Gly Thr Asn Thr Met Ala Thr Gly Ser Gly
        195                 200                 205

Ala Pro Met Ala Asp Asn Asn Glu Gly Ala Asp Gly Val Gly Asn Ser
    210                 215                 220

Ser Gly Asn Trp His Cys Asp Ser Thr Trp Met Gly Asp Arg Val Ile
225                 230                 235                 240

Thr Thr Ser Thr Arg Thr Trp Ala Leu Pro Thr Tyr Asn Asn His Leu
                245                 250                 255

Tyr Lys Gln Ile Ser Ser Gln Ser Gly Ala Ser Asn Asp Asn His Tyr
            260                 265                 270

Phe Gly Tyr Ser Thr Pro Trp Gly Tyr Phe Asp Phe Asn Arg Phe His
        275                 280                 285

Cys His Phe Ser Pro Arg Asp Trp Gln Arg Leu Ile Asn Asn Asn Trp
    290                 295                 300

Gly Phe Arg Pro Lys Arg Leu Asn Phe Lys Leu Phe Asn Ile Gln Val
305                 310                 315                 320

Lys Glu Val Thr Gln Asn Asp Gly Thr Thr Thr Ile Ala Asn Asn Leu
                325                 330                 335

Thr Ser Thr Val Gln Val Phe Thr Asp Ser Glu Tyr Gln Leu Pro Tyr
            340                 345                 350

Val Leu Gly Ser Ala His Gln Gly Cys Leu Pro Pro Phe Pro Ala Asp
        355                 360                 365

Val Phe Met Val Pro Gln Tyr Gly Tyr Leu Thr Leu Asn Asn Gly Ser
    370                 375                 380

Gln Ala Val Gly Arg Ser Ser Phe Tyr Cys Leu Glu Tyr Phe Pro Ser
385                 390                 395                 400

Gln Met Leu Arg Thr Gly Asn Asn Phe Thr Phe Ser Tyr Thr Phe Glu
                405                 410                 415

Asp Val Pro Phe His Ser Ser Tyr Ala His Ser Gln Ser Leu Asp Arg
            420                 425                 430

Leu Met Asn Pro Leu Ile Asp Gln Tyr Leu Tyr Tyr Leu Ser Arg Thr
        435                 440                 445

Asn Thr Pro Ser Gly Thr Thr Thr Gln Ser Arg Leu Gln Phe Ser Gln
    450                 455                 460

Ala Gly Ala Ser Asp Ile Arg Asp Gln Ser Arg Asn Trp Leu Pro Gly
465                 470                 475                 480

Pro Cys Tyr Arg Gln Gln Arg Val Ser Lys Thr Ser Ala Asp Asn Asn
                485                 490                 495

Asn Ser Glu Tyr Ser Trp Thr Gly Ala Thr Lys Tyr His Leu Asn Gly
            500                 505                 510

Arg Asp Ser Leu Val Asn Pro Gly Pro Ala Met Ala Ser His Lys Asp
        515                 520                 525

Asp Glu Glu Lys Phe Phe Pro Gln Ser Gly Val Leu Ile Phe Gly Lys
    530                 535                 540

Gln Gly Ser Glu Lys Thr Asn Val Asp Ile Glu Lys Val Met Ile Thr
545                 550                 555                 560

Asp Glu Glu Glu Ile Arg Thr Thr Asn Pro Val Ala Thr Glu Gln Tyr
                565                 570                 575

Gly Ser Val Ser Thr Asn Leu Gln Arg Gly Gln Arg Gly Asn Arg Gly

580                 585                 590

Thr Glu Trp Asp Ala Gln Ala Ala Thr Ala Asp Val Asn Thr Gln Gly
        595                 600                 605

Val Leu Pro Gly Met Val Trp Gln Asp Arg Asp Val Tyr Leu Gln Gly
    610                 615                 620

Pro Ile Trp Ala Lys Ile Pro His Thr Asp Gly His Phe His Pro Ser
625                 630                 635                 640

Pro Leu Met Gly Gly Phe Gly Leu Lys His Pro Pro Pro Gln Ile Leu
                645                 650                 655

Ile Lys Asn Thr Pro Val Pro Ala Asn Pro Ser Thr Thr Phe Ser Ala
            660                 665                 670

Ala Lys Phe Ala Ser Phe Ile Thr Gln Tyr Ser Thr Gly Gln Val Ser
        675                 680                 685

Val Glu Ile Glu Trp Glu Leu Gln Lys Glu Asn Ser Lys Arg Trp Asn
    690                 695                 700

Pro Glu Ile Gln Tyr Thr Ser Asn Tyr Asn Lys Ser Val Asn Val Asp
705                 710                 715                 720

Phe Thr Val Asp Thr Asn Gly Val Tyr Ser Glu Pro Arg Pro Ile Gly
                725                 730                 735

Thr Arg Tyr Leu Thr Arg Asn Leu
            740

<210>  11
<211>  8359
<212>  DNA
<213>  Adeno-assoziierte Virus 2

<400>  11

catcatcaat aatatacctt attttggatt gaagccaata tgataatgag ggggtggagt        60

ttgtgacgtg gcgcggggcg tgggaacggg gcgggtgacg tagtagctct agaggtcctg       120

tattagaggt cacgtgagtg ttttgcgaca ttttgcgaca ccatgtggtc acgctgggta       180

tttaagcccg agtgagcacg cagggtctcc attttgaagc gggaggtttg aacgcgcagc       240

cgccatgccg gggttttacg agattgtgat taaggtcccc agcgaccttg acgagcatct       300

gcccggcatt tctgacagct ttgtgaactg ggtggccgag aaggaatggg agttgccgcc       360

agattctgac atggatctga atctgattga gcaggcaccc ctgaccgtgg ccgagaagct       420

gcagcgcgac tttctgacgg aatggcgccg tgtgagtaag gcaccggagg cccttttctt 480

tgtgcaattt gagaagggag agagctactt ccacatgcac gtgctcgtgg aaaccaccgg 540

ggtgaaatcc atggttttgg gacgtttcct gagtcagatt cgcgaaaaac tgattcagag 600

aatttaccgc gggatcgagc cgactttgcc aaactggttc gcggtcacaa agaccagaaa 660

tggcgccgga ggcgggaaca aggtggtgga tgagtgctac atccccaatt acttgctccc 720

caaaacccag cctgagctcc agtgggcgtg gactaatatg gaacagtatt taagcgcctg 780

tttgaatctc acggagcgta aacggttggt ggcgcagcat ctgacgcacg tgtcgcagac 840

gcaggagcag aacaaagaga atcagaatcc caattctgat gcgccggtga tcagatcaaa 900

aacttcagcc aggtacatgg agctggtcgg gtggctcgtg gacaagggga ttacctcgga 960

gaagcagtgg atccaggagg accaggcctc atacatctcc ttcaatgcgg cctccaactc 1020

gcggtcccaa atcaaggctg ccttggacaa tgcgggaaag attatgagcc tgactaaaac 1080

cgcccccgac tacctggtgg gccagcagcc cgtggaggac atttccagca atcggattta 1140

taaaattttg gaactaaacg ggtacgatcc ccaatatgcg gcttccgtct ttctgggatg 1200

ggccacgaaa aagttcggca agaggaacac catctggctg tttgggcctg caactaccgg 1260

gaagaccaac atcgcggagg ccatagccca cactgtgccc ttctacgggt gcgtaaactg 1320

gaccaatgag aactttccct tcaacgactg tgtcgacaag atggtgatct ggtgggagga 1380

ggggaagatg accgccaagg tcgtggagtc ggccaaagcc attctcggag gaagcaaggt 1440

gcgcgtggac cagaaatgca agtcctcggc ccagatagac ccgactcccg tgatcgtcac 1500

ctccaacacc aacatgtgcg ccgtgattga cgggaactca acgaccttcg aacaccagca 1560

gccgttgcaa gaccggatgt tcaaatttga actcacccgc cgtctggatc atgactttgg 1620

gaaggtcacc aagcaggaag tcaaagactt tttccggtgg gcaaaggatc acgtggttga 1680

ggtggagcat gaattctacg tcaaaaaggg tggagccaag aaaagacccg cccccagtga 1740

cgcagatata agtgagccca aacgggtgcg cgagtcagtt gcgcagccat cgacgtcaga 1800

cgcggaagct tcgatcaact acgcagacag gtaccaaaac aaatgttctc gtcacgtggg 1860

catgaatctg atgctgtttc cctgcagaca atgcgagaga atgaatcaga attcaaatat 1920

ctgcttcact cacggacaga aagactgttt agagtgcttt cccgtgtcag aatctcaacc 1980

cgtttctgtc gtcaaaaagg cgtatcagaa actgtgctac attcatcata tcatgggaaa 2040

ggtgccagac gcttgcactg cctgcgatct ggtcaatgtg gatttggatg actgcatctt 2100

tgaacaataa atgatttaaa tcaggtatgg ctgccgatgg ttatcttcca gattggctcg 2160

aggacactct ctctgaagga ataagacagt ggtggaagct caaacctggc ccaccaccac 2220

caaagcccgc agagcggcat aaggacgaca gcaggggtct tgtgcttcct gggtacaagt 2280

acctcggacc cttcaacgga ctcgacaagg gagagccggt caacgaggca gacgccgcgg 2340

ccctcgagca cgacaaagcc tacgaccggc agctcgacag cggagacaac ccgtacctca 2400

agtacaacca cgccgacgcg gagtttcagg agcgccttaa agaagatacg tcttttgggg 2460

gcaacctcgg acgagcagtc ttccaggcga aaaagagggt tcttgaacct ctgggcctgg 2520

ttgaggaacc tgttaagacg gctccgggaa aaaagaggcc ggtagagcac tctcctgtgg 2580

agccagactc ctcctcggga accggaaagg cgggccagca gcctgcaaga aaaagattga 2640

attttggtca gactggagac gcagactcag tacctgaccc ccagcctctc ggacagccac 2700

cagcagcccc ctctggtctg ggaactaata cgatggctac aggcagtggc gcaccaatgg 2760

cagacaataa cgagggcgcc gacggagtgg gtaattcctc gggaaattgg cattgcgatt 2820

ccacatggat gggcgacaga gtcatcacca ccagcacccg aacctgggcc ctgcccacct 2880

acaacaacca cctctacaaa caaatttcca gccaatcagg agcctcgaac gacaatcact 2940

actttggcta cagcacccct tgggggtatt ttgacttcaa cagattccac tgccactttt 3000

caccacgtga ctggcaaaga ctcatcaaca acaactgggg attccgaccc aagagactca 3060

acttcaagct ctttaacatt caagtcaaag aggtcacgca gaatgacggt acgacgacga 3120

ttgccaataa ccttaccagc acggttcagg tgtttactga ctcggagtac cagctcccgt 3180

acgtcctcgg ctcggcgcat caaggatgcc tcccgccgtt cccagcagac gtcttcatgg 3240

tgccacagta tggatacctc accctgaaca acgggagtca ggcagtagga cgctcttcat 3300

tttactgcct ggagtacttt ccttctcaga tgctgcgtac cggaaacaac tttaccttca 3360

gctacacttt tgaggacgtt cctttccaca gcagctacgc tcacagccag agtctggacc 3420

gtctcatgaa tcctctcatc gaccagtacc tgtattactt gagcagaaca aacactccaa 3480

gtggaaccac cacgcagtca aggcttcagt tttctcaggc cggagcgagt gacattcggg 3540

accagtctag gaactggctt cctggaccct gttaccgcca gcagcgagta tcaaagacat 3600

ctgcggataa caacaacagt gaatactcgt ggactggagc taccaagtac cacctcaatg 3660

gcagagactc tctggtgaat ccgggcccgg ccatggcaag ccacaaggac gatgaagaaa 3720

agttttttcc tcagagcggg gttctcatct ttgggaagca aggctcagag aaaacaaatg 3780

tggacattga aaaggtcatg attacagacg aagaggaaat caggacaacc aatcccgtgg 3840

ctacggagca gtatggttct gtatctacca acctccagag aggccagaga ggcaatcggg 3900

ggactgagtg ggatgcccag gcggccaccg cagatgtcaa cacacaaggc gttcttccag 3960

gcatggtctg gcaggacaga gatgtgtacc ttcaggggcc catctgggca aagattccac 4020

acacggacgg acattttcac ccctctcccc tcatgggtgg attcggactt aaacaccctc 4080

ctccacagat tctcatcaag aacaccccgg tacctgcgaa tccttcgacc accttcagtg 4140

cggcaaagtt tgcttccttc atcacacagt actccacggg acaggtcagc gtggagatcg 4200

agtgggagct gcagaaggaa aacagcaaac gctggaatcc cgaaattcag tacacttcca 4260

actacaacaa gtctgttaat gtggacttta ctgtggacac taatggcgtg tattcagagc 4320

ctcgccccat tggcaccaga tacctgactc gtaatctgta attgcttgtt aatcaataaa 4380

ccgtttaatt cgtttcagtt gaactttggt ctctgcgtat ttctttctta tctagtttcc 4440

atgctctaga ggtcctgtat tagaggtcac gtgagtgttt tgcgacattt tgcgacacca 4500

tgtggtcacg ctgggtattt aagcccgagt gagcacgcag ggtctccatt ttgaagcggg 4560

aggtttgaac gcgcagccac cacggcgggg ttttacgaga ttgtgattaa ggtccccagc 4620

gaccttgacg agcatctgcc cggcatttct gacagctttg tgaactgggt ggccgagaag 4680

gaatgggagt tgccgccaga ttctgacatg gatctgaatc tgattgagca ggcacccctg 4740

accgtggccg agaagctgca tcgctggcgt aatagcgaag aggcccgcac cgatcgccct 4800

tcccaacagt tgcgcagcct gaatggcgaa tggcgattcc gttgcaatgg ctggcggtaa 4860

tattgttctg gatattacca gcaaggccga tagtttgagt tcttctactc aggcaagtga 4920

tgttattact aatcaaagaa gtattgcgac aacggttaat ttgcgtgatg gacagactct 4980

tttactcggt ggcctcactg attataaaaa cacttctcag gattctggcg taccgttcct 5040

gtctaaaatc cctttaatcg gcctcctgtt tagctcccgc tctgattcta acgaggaaag 5100

cacgttatac gtgctcgtca aagcaaccat agtacgcgcc ctgtagcggc gcattaagcg 5160

cggcgggtgt ggtggttacg cgcagcgtga ccgctacact tgccagcgcc ctagcgcccg 5220

ctcctttcgc tttcttccct tcctttctcg ccacgttcgc cggctttccc cgtcaagctc 5280

taaatcgggg gctcccttta gggttccgat ttagtgcttt acggcacctc gaccccaaaa 5340

aacttgatta gggtgatggt tcacgtagtg ggccatcgcc ctgatagacg gtttttcgcc 5400

ctttgacgtt ggagtccacg ttctttaata gtggactctt gttccaaact ggaacaacac 5460

tcaaccctat ctcggtctat tcttttgatt tataagggat tttgccgatt tcggcctatt 5520

ggttaaaaaa tgagctgatt taacaaaaat ttaacgcgaa ttttaacaaa atattaacgt 5580

ttacaattta aatatttgct tatacaatct tcctgttttt ggggcttttc tgattatcaa 5640

ccggggtaca tatgattgac atgctagttt tacgattacc gttcatcgat tctcttgttt 5700

gctccagact ctcaggcaat gacctgatag cctttgtaga gacctctcaa aaatagctac 5760

cctctccggc atgaatttat cagctagaac ggttgaatat catattgatg gtgatttgac 5820

tgtctccggc ctttctcacc cgtttgaatc tttacctaca cattactcag gcattgcatt 5880

taaaatatat gagggttcta aaaattttta tccttgcgtt gaaataaagg cttctcccgc 5940

aaaagtatta cagggtcata atgtttttgg tacaaccgat ttagctttat gctctgaggc 6000

tttattgctt aattttgcta attctttgcc ttgcctgtat gatttattgg atgttggaat 6060

tcctgatgcg gtattttctc cttacgcatc tgtgcggtat ttcacaccgc atatggtgca 6120

ctctcagtac aatctgctct gatgccgcat agttaagcca gccccgacac ccgccaacac 6180

ccgctgacgc gccctgacgg gcttgtctgc tcccggcatc cgcttacaga caagctgtga 6240

ccgtctccgg gagctgcatg tgtcagaggt tttcaccgtc atcaccgaaa cgcgcgagac 6300

gaaagggcct cgtgatacgc ctatttttat aggttaatgt catgataata atggtttctt 6360

agacgtcagg tggcactttt cggggaaatg tgcgcggaac ccctatttgt ttatttttct 6420

aaatacattc aaatatgtat ccgctcatga gacaataacc ctgataaatg cttcaataat 6480

attgaaaaag gaagagtatg agtattcaac atttccgtgt cgcccttatt cccttttttg 6540

cggcattttg ccttcctgtt tttgctcacc cagaaacgct ggtgaaagta aaagatgctg 6600

aagatcagtt gggtgcacga gtgggttaca tcgaactgga tctcaacagc ggtaagatcc 6660

ttgagagttt tcgccccgaa gaacgttttc caatgatgag cacttttaaa gttctgctat 6720

gtggcgcggt attatcccgt attgacgccg ggcaagagca actcggtcgc cgcatacact 6780

attctcagaa tgacttggtt gagtactcac cagtcacaga aaagcatctt acggatggca 6840

tgacagtaag agaattatgc agtgctgcca taaccatgag tgataacact gcggccaact 6900

tacttctgac aacgatcgga ggaccgaagg agctaaccgc ttttttgcac aacatggggg 6960

atcatgtaac tcgccttgat cgttgggaac cggagctgaa tgaagccata ccaaacgacg 7020

agcgtgacac cacgatgcct gtagcaatgg caacaacgtt gcgcaaacta ttaactggcg 7080

aactacttac tctagcttcc cggcaacaat taatagactg gatggaggcg gataaagttg 7140

caggaccact tctgcgctcg gcccttccgg ctggctggtt tattgctgat aaatctggag 7200

ccggtgagcg tgggtctcgc ggtatcattg cagcactggg gccagatggt aagccctccc 7260

gtatcgtagt tatctacacg acggggagtc aggcaactat ggatgaacga aatagacaga 7320

tcgctgagat aggtgcctca ctgattaagc attggtaact gtcagaccaa gtttactcat 7380

atatacttta gattgattta aaacttcatt tttaatttaa aaggatctag gtgaagatcc 7440

tttttgataa tctcatgacc aaaatccctt aacgtgagtt ttcgttccac tgagcgtcag 7500

accccgtaga aaagatcaaa ggatcttctt gagatccttt ttttctgcgc gtaatctgct 7560

gcttgcaaac aaaaaaacca ccgctaccag cggtggtttg tttgccggat caagagctac 7620

caactctttt tccgaaggta actggcttca gcagagcgca gataccaaat actgtccttc 7680

tagtgtagcc gtagttaggc caccacttca agaactctgt agcaccgcct acatacctcg 7740

ctctgctaat cctgttacca gtggctgctg ccagtggcga taagtcgtgt cttaccgggt 7800

tggactcaag acgatagtta ccggataagg cgcagcggtc gggctgaacg gggggttcgt 7860

gcacacagcc cagcttggag cgaacgacct acaccgaact gagataccta cagcgtgagc 7920

tatgagaaag cgccacgctt cccgaaggga gaaaggcgga caggtatccg gtaagcggca 7980

gggtcggaac aggagagcgc acgagggagc ttccaggggg aaacgcctgg tatctttata 8040

gtcctgtcgg gtttcgccac ctctgacttg agcgtcgatt tttgtgatgc tcgtcagggg 8100

ggcggagcct atggaaaaac gccagcaacg cggccttttt acggttcctg gccttttgct 8160

ggccttttgc tcacatgttc tttcctgcgt tatcccctga ttctgtggat aaccgtatta 8220

ccgcctttga gtgagctgat accgctcgcc gcagccgaac gaccgagcgc agcgagtcag 8280

tgagcgagga agcggaagag cgcccaatac gcaaaccgcc tctccccgcg cgttggccga 8340

ttcattaatg cagaattcc 8359

<210> 12
<211> 56
<212> DNA
<213> Künstliche Sequenz

<220>
<223> PCR Primer

<220>
<221> misc_feature
<222> (18)..(38)
<223> n ist a, c, g, oder t

<400> 12

cagtcggcca gagaggcnnn nnnnnnnnnn nnnnnnnngc ccaggcggct gacgag 56

<210> 13
<211> 16
<212> DNA
<213> Künstliche Sequenz

<220>
<223> PCR Primer

<400> 13

ctcgtcagcc gcctgg 16

<210> 14
<211> 26
<212> DNA
<213> Künstliche Sequenz

<220>
<223> PCR Primer

<400> 14

gcagtatggt tctgtatcta ccaacc 26

<210> 15
<211> 22

<212>  DNA
<213>  Künstliche Sequenz

<220>
<223>  PCR Primer

<400>  15

gcctggaaga acgccttgtg tg                                                22


<210>  16
<211>  22
<212>  DNA
<213>  Künstliche Sequenz

<220>
<223>  PCR Primer

<400>  16

atggcaagcc acaaggacga tg                                                22


<210>  17
<211>  22
<212>  DNA
<213>  Künstliche Sequenz

<220>
<223>  PCR Primer

<400>  17

cgtggagtac tgtgtgatga ag                                                22


<210>  18
<211>  23
<212>  DNA
<213>  Künstliche Sequenz

<220>
<223>  PCR Primer

<400>  18

ggttctcatc tttgggaagc aag                                               23


<210>  19
<211>  21
<212>  DNA
<213>  Künstliche Sequenz

<220>
<223>  PCR Primer

<400>  19

tgatgagaat ctgtggagga g                                                 21

<210>  20
<211>  19
<212>  DNA
<213>  Künstliche Sequenz

<220>
<223>  PCR Primer

<400>  20

ggcggagttg ttacgacat                                                    19


<210>  21
<211>  20
<212>  DNA
<213>  Künstliche Sequenz

<220>
<223>  PCR Primer

<400>  21

gggactttcc ctacttggca                                                   20


<210>  22
<211>  7
<212>  PRT
<213>  Künstliche Sequenz

<220>
<223>  Gehirnspezifisches Peptid

<400>  22

Gly Trp Gln Pro Gln Val Gln
1               5


<210>  23
<211>  7
<212>  PRT
<213>  Künstliche Sequenz

<220>
<223>  Gehirnspezifisches Peptid

<400>  23

Gly Ser Asp Lys Gly Glu Ser
1               5


<210>  24
<211>  7
<212>  PRT
<213>  Künstliche Sequenz

<220>
<223>  Gehirnspezifisches Peptid

<400>  24

Gly Thr Asp Gly Gly Glu Asn
1               5


<210>  25
<211>  7
<212>  PRT
<213>  Künstliche Sequenz

<220>
<223>  Gehirnspezifisches Peptid

<400>  25

Asn Ala Glu Arg Gly Glu Gly
1               5


<210>  26
<211>  7
<212>  PRT
<213>  Künstliche Sequenz

<220>
<223>  Gehirnspezifisches Peptid

<400>  26

Asn Asn Ala Thr Arg Asp Trp
1               5


<210>  27
<211>  7
<212>  PRT
<213>  Künstliche Sequenz

<220>
<223>  Gehirnspezifisches Peptid

<400>  27

Asn Ser Ala Ala Arg Ile Glu
1               5


<210>  28
<211>  7
<212>  PRT
<213>  Künstliche Sequenz

<220>
<223>  Gehirnspezifisches Peptid

<400>  28

Asn Ser Ala Ala Arg Trp Glu
1               5


<210>  29
<211>  7

<212>  PRT
<213>  Künstliche Sequenz

<220>
<223>  Gehirnspezifisches Peptid

<400>  29

Lys Asp Ala Trp Glu Arg Glu
1               5


<210>  30
<211>  7
<212>  PRT
<213>  Künstliche Sequenz

<220>
<223>  Gehirnspezifisches Peptid

<400>  30

Lys Lys Gln Gln Trp Asp Gln
1               5


<210>  31
<211>  7
<212>  PRT
<213>  Künstliche Sequenz

<220>
<223>  Gehirnspezifisches Peptid

<400>  31

Ala His Gly Ala Glu Gln Ala
1               5


<210>  32
<211>  7
<212>  PRT
<213>  Künstliche Sequenz

<220>
<223>  Gehirnspezifisches Peptid

<400>  32

Asn Arg Thr Ala Asp Thr Gly
1               5


<210>  33
<211>  7
<212>  PRT
<213>  Künstliche Sequenz

<220>
<223>  Gehirnspezifisches Peptid

<400>  33

Asn Ser Gly Tyr Gly Tyr Thr
1               5

<210>  34
<211>  28
<212>  PRT
<213>  Künstliche Sequenz

<220>
<223>  Gehirnspezifisches Peptid

<400>  34

Asn Asn Val Arg Thr Ser Glu Asn Asn Val Arg Thr Ser Glu Asn Asn
1               5                   10                  15

Val Arg Thr Ser Glu Asn Asn Val Arg Thr Ser Glu
            20                  25

<210>  35
<211>  7
<212>  PRT
<213>  Künstliche Sequenz

<220>
<223>  Gehirnspezifisches Peptid

<400>  35

Asn Glu Ser Arg Arg Leu Glu
1               5

<210>  36
<211>  7
<212>  PRT
<213>  Künstliche Sequenz

<220>
<223>  Gehirnspezifisches Peptid

<400>  36

Gly Gly Gly Lys Arg Arg Glu
1               5

<210>  37
<211>  7
<212>  PRT
<213>  Künstliche Sequenz

<220>
<223>  Gehirnspezifisches Peptid

<400>  37

Glu Gly Asp Ile Arg Trp Val
1               5

<210>  38
<211>  7
<212>  PRT
<213>  Künstliche Sequenz

<220>
<223>  Gehirnspezifisches Peptid

<400>  38

Ser Asn Tyr Ser Arg Val Met
1               5


<210>  39
<211>  14
<212>  PRT
<213>  Künstliche Sequenz

<220>
<223>  Gehirnspezifisches Peptid

<400>  39

Asn Asn Val Arg Thr Ser Glu Asn Asn Val Arg Thr Ser Glu
1               5                   10


<210>  40
<211>  7
<212>  PRT
<213>  Künstliche Sequenz

<220>
<223>  Gehirnspezifisches Peptid

<400>  40

Asn Met Ala Arg Gln Ala Asp
1               5


<210>  41
<211>  56
<212>  PRT
<213>  Künstliche Sequenz

<220>
<223>  Gehirnspezifisches Peptid

<400>  41

Ser Asp Gly Leu Thr Trp Ser Ser Asp Gly Leu Thr Trp Ser Ser Asp
1               5                   10                  15


Gly Leu Thr Trp Ser Ser Asp Gly Leu Thr Trp Ser Ser Asp Gly Leu
            20                  25                  30


Thr Trp Ser Ser Asp Gly Leu Thr Trp Ser Ser Asp Gly Leu Thr Trp

        35                  40                  45

Ser Ser Asp Gly Leu Thr Trp Ser
    50                  55

<210> 42
<211> 7
<212> PRT
<213> Künstliche Sequenz

<220>
<223> Gehirnspezifisches Peptid

<400> 42

Asp Asp Gly Val Ser Trp Lys
1               5

## Beschreibung

Die Erfindung betrifft neuartige Peptide, Polypeptide oder Proteine, die spezifisch an Zellen des Gehirns und/oder des Rückenmarks binden. Die Peptide, Polypeptide oder Proteine können Bestandteil eines viralen Kapsids sein und dazu verwendet werden, einen rekombinanten viralen Vektor nach systemischer Verabreichung an ein Subjekt selektiv zum Gehirn und/oder zum Rückenmark zu leiten und dort für eine gewebespezifische Expression von einem oder mehreren Transgenen zu sorgen. Die Erfindung betrifft somit auch einen rekombinanten viralen Vektor, vorzugsweise einen AAV-Vektor, der ein Kapsid umfasst, welches mindestens eines der erfindungsgemäßen Peptide, Polypeptide oder Proteine enthält, sowie mindestens ein in dem Kapsid verpacktes Transgen. Der virale Vektor eignet sich insbesondere zur therapeutischen Behandlung einer Erkrankung oder Funktionsstörung des Gehirns und/oder des Rückenmarks. Die Erfindung betrifft ferner Zellen und pharmazeutische Zusammensetzungen, die den erfindungsgemäßen viralen Vektor umfassen.

### HINTERGRUND DER ERFINDUNG

Die gentherapeutische Behandlung mittels viraler Vektoren stellt eine vielversprechende Behandlungsmöglichkeit für Erkrankungen dar, die auf eine konventionelle Behandlung nicht oder nicht in ausreichendem Maße ansprechen. Dieser Ansatz beruht auf der Einschleusung von therapeutischen Genen in den zu behandelnden Organismus mit Hilfe von Viren, die so modifizierte wurden, dass sie die Sequenz des entsprechenden Gens in ihrem Genom aufweisen. Virale Vektoren, die im Rahmen der Gentherapie bereits für gentherapeutische Therapieansätze verwendet wurden, basieren auf Retroviren, Lentiviren, Adenoviren und Adeno-assoziierte Viren.

Adeno-assozierte Viren (AAV) stellen vielversprechende Kandidaten für den Einsatz in der der klinischen Praxis dar, da sie als verhältnismäßig sicher eingestuft werden. AAV-Vektoren sind in der Lage, ein Transgen in ein Gewebe einzuschleusen und dort stabil und effizient zu exprimieren. Gleichzeitig weisen diese Vektoren keine bekannten Pathogenitätsmechanismen auf [1]. Besondere Bedeutung für eine klinische Anwendung kommt den AAV-Vektoren vom Serotyp 2 (AAV2) zu, die als besonders gut untersucht gelten. Nach Einschleusung durch AAV-Vektoren können die Transgene in verschiedenen Formen in der transfizierten Zelle vorliegen, z.B. als episomale, einzel- oder doppelsträngige DNA. Auch konkatamere Formen der DNA wurden in transduzierten Zellen nachgewiesen.

Das Genom von AAV2 wird von einem linearen, einzelsträngigen DNA-Molekül von etwa 4700 Nukleotiden Länge gebildet und umfasst an beiden Enden Inverted Terminal Repeats (ITRs). Das Genom umfasst ferner zwei große offene Leserahmen, die als Replikations-Region (rep) und Kapsid-Region (cap) bezeichnet werden. Die Replikations-Region kodiert für Proteine, die im Rahmen der Virusreplikation erforderlich sind. Die Kapsid-Region hingegen kodiert für die strukturellen Proteine VP1, VP2 und VP3, aus denen sich das ikosaedrische Kapsid des Virus zusammensetzt.

Wie die meisten im Stand der Technik bekannten, gentherapeutisch verwendbaren Vektoren allerdings weisen Wildtyp-AAV-Vektoren, wie z.B. die beschriebenen AAV2-Vektoren, keine ausreichende Spezifität für ein bestimmtes Gewebe auf, sondern infizieren eine große Bandbreite von Zelltypen. Bei systemischer Gabe dieser Wildtyp-Vektoren kommt es somit zu einer unzureichenden Transduktion der Zielgewebe, während aufgrund der unerwünschten Transduktion von anderen Geweben mit starken Immunreaktionen im behandelten Patienten gerechnet werden muss. Fortschritte bei der Entwicklung von viralen Vektoren, die eine erhöhte Spezifität für bestimmte Organe aufweisen, wurden in der Vergangenheit durch den Einsatz von Peptidliganden erreicht, die in der Lage sind, die Vektoren zu einem bestimmten Organ zu leiten [2-3]. Es konnte gezeigt werden, dass bestimmte Peptidliganden für ein "Homing" zu verschiedenen Organen wie z.B. zum Gehirn sorgen.

Literaturstelle [4] beschreibt ein Verfahren, welches das Screening nach Kapsiden von AAV2 mit modifiziertem Tropismus in randomisierten Peptidbanken erlaubt. Aus diesen Bibliotheken können Vektoren isoliert werden, die *in vitro* spezifisch einen gewünschten Zelltyp transduzieren. Jedoch wurde festgestellt, dass die auf diese Weise selektierten Kapside oftmals für eine Verwendung *in vivo* ungeeignet sind, da ihnen im Tiermodell die nötige Spezifität fehlte [5].

Das Gehirn stellt ein klinisch extrem relevantes Organ dar, da es Ausgangspunkt für eine Reihe von neurologischen Erkrankungen ist. Es wurden erhebliche Anstrengungen unternommen, dieses Organ für gentherapeutische Interventionen erreichbar zu machen [6-12]. Allerdings stellt die Blut-Hirn-Schranke, die gemeinschaftlich von Endothelzellen, Perizyten und Astrozyten gebildet wird und das Gehirn von zirkulierenden Partikeln, Toxinen und Botenstoffen abschottet, ein für gängige Vektorsysteme unüberwindbares Hindernis dar. Da bislang keine geeigneten Vektorsysteme verfügbar sind, die nach intravenöser Applikation zielgerichtet mit ausreichender Effizienz das Gehirn transduzieren, werden heutige Gentherapievektoren meist direkt in das Gehirn injiziert [13], was mit einem hohen Risiko für den Patienten behaftet ist. Die Möglichkeit, die Blut-Hirn-Schranke kurzzeitig für größere Partikel passierbar zu machen, z.B. durch Ultraschall [14] oder mittels chemischer Komponenten [7], wird ebenfalls als sehr riskant erachtet.

Es besteht daher ein großes Bedürfnis nach Mitteln, die in der Lage sind, den Tropismus viraler Vektoren zu modulieren und damit für eine ausreichende Zell- oder Gewebespezifität sorgen, die einen gezielten Transport eines viralen Vektors zu Geweben des Gehirn und Rückenmarks, ermöglicht. Solche Vektoren können für eine spezifische Expression von therapeutischen Genen in diesen Geweben sorgen, um auf diese Weise Erkrankungen und/oder Funktionsstörungen des Gehirns und Rückenmarks wirksam zu behandeln.

Die vorliegende Erfindung stellt virale Vektoren für den zielgerichteten Gentransfer in das Gehirn und Rückenmarks bereit. Die erfindungsgemäßen viralen Vektoren exprimieren auf ihrer Kapsid-Oberfläche bislang unbekannte Aminosäuresequenzen, die *in vivo* spezifisch von Rezeptoren auf den Neuronen des Gehirns und den Endothelzellen der Blutgefäße von Gehirn und Rückenmark erkannt werden. Somit transduzieren die viralen Vektoren der vorliegenden Erfindung nach systemischer Verabreichung an ein Subjekt spezifisch dessen Gewebe in Gehirn und Rückenmark.

Die erfindungsgemäßen viralen Vektoren ermöglichen darüber hinaus eine starke und lang anhaltende Expression eines Transgens in den Neuronen des Gehirns und den endothelialen Zellen der Blutgefäße von Gehirn und Rückenmark. Daher sind die Vektoren insbesondere für die gentherapeutische Behandlung von bestimmten Erkrankungen und Funktionsstörungen von Gehirn und Rückenmark geeignet. Es wurde darüber hinaus festgestellt, dass die die AAV-Vektoren nach der Transfektion lediglich eine geringfügige Immunreaktion in dem Wirt hervorrufen und somit besonders für die Gentherapie geeignet sind.

### BESCHREIBUNG DER ERFINDUNG

Im Rahmen der vorliegenden Erfindung wurden durch Selektion in einer randomisierten AAV2-Heptamer-Pepidbibliothek verschiedene für das Zentralnervensystem, d.h. für Gehirn und Rückenmark, spezifische Sequenzen identifiziert. Ein Heptamer, das eine besondere Spezifität für das Gehirn aufwies, war das Peptid NRGTEWD (SEQ ID NO:1). Auf Basis dieser Sequenz wurde der rekombinante virale Vektor rAAV2-NRGTEWD hergestellt, bei dem die Peptidsequenz NRGTEWD als Teilsequenz des Kapsid-Proteins VP1 exprimiert wird. Anschließend wurde der Vektor rAAV2- NRGTEWD intravenös an Mäuse verabreicht, wobei sowohl *in vitro* als auch *in vivo* eine eindeutige Spezifität des Vektors für das Zentralnervensystem beobachtet werden konnte. Sowohl Neuronen des Gehirns als auch Endothelzellen der Blutgefäße von Gehirn und Rückenmark wurden von dem Vektor transfiziert. Die Spezifität konnte durch Färbung mit CD31 nachgewiesen werden, wie es in den vorliegenden Beispielen beschrieben wird.

Eine weitere Gruppe von Peptiden, die ebenfalls eine Spezifität für Gehirn und Rückenmark zeigten, umfasste die Peptide ADGVQWT (SEQ ID NO:2), DDGVSWK (SEQ ID NO:3), SDGLTWS (SEQ ID NO:4) und SDGLAWV (SEQ ID NO:5). Diese Peptide enthielten jeweils das allgemeine Motiv XDGXXWX (SEQ ID NO:6).

Die vorliegende Erfindung stellt daher verschiedene für das Gehirn und das Rückenmark spezifische Peptidsequenzen bereit, die in besonderer Weise dazu geeignet sind, therapeutische Mittel, wie z.B. virale Vektoren, gezielt in das Gehirn bzw. das Rückenmark eines zu behandelnden Subjekts zu leiten.

In einem ersten Aspekt betrifft die vorliegende Erfindung demgemäß ein Peptid, Polypeptid oder Protein, das spezifisch an Zellen des Gehirns und/oder des Rückenmarks bindet, wobei das Peptid, Polypeptid oder Protein die Aminosäuresequenz von SEQ ID NO:1 oder eine Variante davon umfasst, wobei sich die Variante von der Aminosäuresequenz von SEQ ID NO:1 durch Modifikation von maximal einer Aminosäure unterscheidet. Das erfindungsgemäße Peptid, Polypeptid oder Protein bindet vorzugsweise an Endothelzellen und/oder an Neuronen des Gehirns oder Rückenmarks.

In einem zweiten Aspekt betrifft die vorliegende Erfindung ein Peptid, Polypeptid oder Protein, das spezifisch an Zellen des Gehirns und/oder des Rückenmarks bindet, wobei das Peptid, Polypeptid oder Protein die allgemeine Aminosäuresequenz von SEQ ID NO:6 umfasst. In einer besonders bevorzugten Ausführungsform umfasst das Peptid, Polypeptid oder Protein eine der Sequenzen ADGVQWT (SEQ ID NO:2), DDGVSWK (SEQ ID NO:3), SDGLTWS (SEQ ID NO:4) oder SDGLAWV (SEQ ID NO:5) oder eine Variante derselben, wobei sich die Variante von der jeweiligen Aminosäuresequenz von SEQ ID NO:2-5 durch Modifikation von maximal einer Aminosäure unterscheidet. Das erfindungsgemäße Peptid, Polypeptid oder Protein bindet vorzugsweise an Endothelzellen und/oder an Neuronen des Gehirns oder Rückenmarks.

Im Rahmen der vorliegenden Offenbarung bezeichnet der Begriff "Peptid" eine Verknüpfung von 2-10 Aminosäuren, die über eine peptidische Bindung miteinander verbunden sind. Der Begriff "Polypeptid" bezeichnet eine Verknüpfung von 11-100 Aminosäuren, die über eine peptidische Bindung miteinander verbunden sind. Polypeptide mit mehr als 100 Aminosäuren werden vorliegend als "Protein" bezeichnet.

In einer besonders bevorzugten Ausführungsform der Erfindung ist die erfindungsgemäße, für das Gehirns und das Rückenmark spezifische Peptidsequenz gemäß SEQ ID NO:1 oder einer Variante davon oder gemäß SEQ ID NO:2-6 oder einer Variante davon Teil eines Kapsid-Proteins eines Virus. Dies bedeutet, dass die für das Gehirn und das Rückenmark spezifische Peptidse-quenz als Teil eines Kapsid-Proteins des Virus vorliegt. Um derartige Kapsid-Proteine herzustellen, wird eine entsprechende, für das Peptid kodierende Nukleotidsequenz in die Region des Virus-Genoms kloniert, die für ein Kapsid-Protein des Virus kodiert. Wird die für das Gehirn bzw. das Rückenmark spezifische Sequenz als Teil eines Kapsid-Proteins exprimiert, so kann sie vielfach an der Oberfläche des viralen Vektors präsentiert werden.

Vorzugsweise handelt es sich bei dem Kapsid-Protein um ein solches, das von einem Adeno-assoziierten Virus (AAV) stammt. Bei dem AAV kann es sich um einen beliebigen der im Stand der Technik beschriebenen Serotypen handeln, wobei das Kapsid-Protein vorzugsweise von einem AAV von einem der Serotypen 2, 4, 6, 8 und 9 stammt. Ein Kapsid-Protein eines AAV vom Serotyp 2 ist besonders bevorzugt.

Das Kapsid des AAV-Wildtyps setzt sich aus den Kapsid-Proteinen VP1, VP2 und VP3 zusammen, die überlappend von der cap-Region kodiert werden. Alle drei Proteine weisen einen identischen C-terminalen Bereich auf. Das Kapsid von AAV umfasst etwa 60 Kopien der Proteine VP1, VP2 und VP3, die im Verhältnis 1:1:8 exprimiert werden. Wird eine Nukleotidsequenz, die für eine der vorliegend beschriebenen Peptide mit Spezifität für das Gehirn bzw. das Rückenmark kodiert, C-terminal in den Leserahmen von VP1 kloniert (d.h. in den Bereich, der bei allen drei Proteinen identisch ist), so kann man erwarten, dass theoretisch 60 der spezifischen Peptide der auf der Kapsid-Oberfläche zu finden sind.

Wird ein AAV-Vektor im Sinne der vorliegenden Erfindung modifiziert, so wird die für das Peptid mit Spezifität für das Gehirn kodierende Nukleotidsequenz in die cap-Region am 3'-Ende des Genoms kloniert. Die Sequenz, die für das Peptid mit Spezifität für das Gehirn bzw. Rückenmark kodiert, kann in die genomische Sequenz eines der Kapsid-Proteine VP1, VP2 oder VP3 kloniert werden. Die Kapsid-Proteine von AAV2 sind beispielhaft in SEQ ID NO:7 (VP1), SEQ ID NO:8 (VP2) und SEQ ID NO:9 (VP3) dargestellt.

In einer besonders bevorzugten Ausführungsform der Erfindung wird die Sequenz, die für das Peptid kodiert, welches Spezifität für das Gehirn bzw. Rückenmark aufweist, in den Leserahmen eines VP1-Gens kloniert, vorzugsweise in das in SEQ ID NO:7 gezeigte VP1-Gen von AAV2. Dabei sollte beachtet werden, dass sich durch die Insertierung der klonierten Sequenz kein Wechsel des Leserahmens und kein vorzeitiger Abbruch der Translation ergibt. Dem Fachmann werden die dazu erforderlichen Methoden ohne Weiteres ersichtlich sein.

In allen drei Kapsid-Proteinen von AVV wurden Stellen identifiziert, an denen Peptidsequenzen für das Homing eingefügt werden können [15-20]. So wurde unter anderem das im VP1 von AAV2 vorkommende Arginin in Position 588 (R588) spezifisch für die Insertion eines Peptidliganden vorgeschlagen [21-22]. Diese Aminosäureposition des viralen Kapsids ist offenbar an der Bindung von AAV2 an seinen natürlichen Rezeptor beteiligt. Es wurde vermutet, dass R588 einer von vier Arginin-Resten ist, der die Bindung von AAV2 an seinen natürlichen Rezeptor vermittelt [23-24]. Eine Modifikation in diesem Bereich des Kapsids schwächt den natürlichen Tropismus von AAV2 ab oder beseitigt diesen vollständig.

Demgemäß ist es erfindungsgemäß besonders bevorzugt, dass die erfindungsgemäße Peptidsequenz gemäß SEQ ID NO:1 oder einer Variante davon oder gemäß SEQ ID NO:2-6 oder einer Variante davon im Bereich der Aminosäuren 550-600 des VP1-Proteins von AAV2, insbesondere des VP1-Proteins von SEQ ID NO:7, insertiert vorliegt. Noch stärker bevorzugt ist es, dass die Peptidsequenz im Bereich der Aminosäuren 560-600, 570-600, 560-590, 570-590 des VP1-Proteins inseriert vorliegt.

So kann sich die Peptidsequenz gemäß SEQ ID NO:1 oder einer Variante davon oder gemäß SEQ ID NO:2-6 oder einer Variante davon z.B. unmittelbar hinter einer der folgenden Aminosäure des VP1-Proteins, insbesondere des Proteins von SEQ ID NO:7, anschließen: 550, 551, 552, 553, 554, 555, 556, 557, 558, 559, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599 oder 600.

Besonders bevorzugt ist es, dass die Peptidsequenz gemäß SEQ ID NO:1 oder einer Variante davon oder gemäß SEQ ID NO:2-6 oder einer Variante davon auf die Aminosäure 588 des VP1-Proteins von SEQ ID NO:7 folgt, wie es in den Beispielen gezeigt ist. Es ist dabei möglich, dass zwischen dem Arginin-Rest in Position 588 und der ersten Aminosäure des erfindungsgemäßen Peptids oder der Variante davon eine oder mehrere, und insbesondere bis zu 5 (d.h. 1, 2, 3, 4 oder 5) Aminosäuren liegen, die durch die Klonierung bedingt sind. Ebenso können hinter der letzten Aminosäure des erfindungsgemäßen Peptids oder der Variante davon eine oder mehrere, insbesondere bis zu 5 (d.h. 1, 2, 3, 4 oder 5) Aminosäuren liegen.

Die oben in Bezug auf VP1 angegebenen Stellen und Bereiche in der Aminosäuresequenz des Kapsid-Proteins gelten analog auch für die Kapsid-Proteine VP2 und VP3 von AAV2. Da sich die drei Kapsid-Proteine VP1, VP2 und VP3 von AAV2 lediglich durch die Länge der N-terminalen Sequenz unterscheiden und demgemäß einen identischen C-terminalen Bereich aufweisen, wird der Fachmann keine Probleme haben, durch Sequenzvergleich die oben für VP1 angegebenen Stellen für die Insertierung des Peptidliganden in den Aminosäuresequenzen von VP1 und VP2 zu identifizieren. So entspricht die Aminosäure R588 in VP1 der Position R451 von VP2 (SEQ ID NO:8) bzw. der Position R386 von VP3 (SEQ ID NO:9).

SEQ ID NO:10 zeigt beispielhaft die Sequenz des VP1-Proteins von AVV2 nach Einbringung der Peptidsequenz von SEQ ID NO:1. Bedingt durch die Klonierung weist das Kapsid-Protein zwei zusätzliche Aminosäuren auf, die in der nativen Sequenz des VP1-Proteins von AVV2 nicht vorkommen. So wird die Peptidsequenz von SEQ ID NO:1 N-terminal von einem Glycin in Position 589 und C-terminal von einem Alanin in Position 597 flankiert. Darüber hinaus ist das Asparagin in Position 587 der nativen Sequenz gegen ein Glutamin ausgetauscht.

In einer besonderen Ausführungsform betrifft die vorliegende Erfindung somit auch ein Kapsid-Protein, das Folgendes umfasst oder daraus besteht:
(a) die Aminosäuresequenz von SEQ ID NO:10;
(b) eine Aminosäuresequenz, die mindestens 80% und vorzugsweise 90, 95 oder 99% Identität zu der Aminosäuresequenz von SEQ ID NO:10 aufweist und darüber hinaus (i) die Sequenz von SEQ ID NO:1 umfasst oder (ii) eine Aminosäuresequenz, die sich von der Aminosäuresequenz von SEQ ID NO:1 durch Modifikation von einer Aminosäure unterscheidet; oder
(c) ein Fragment von einer der in (a) oder (b) definierten Aminosäuresequenzen.

In einem weiteren Aspekt richtet sich die Erfindung auf ein virales Kapsid, das ein Peptid, Polypeptid oder Protein umfasst, welches spezifisch an Zellen des Gehirns bzw. des Rückenmarks bindet und die Peptidsequenz gemäß SEQ ID NO:1 oder einer Variante davon oder gemäß SEQ ID NO:2-6 oder einer Variante davon umfasst.

Des Weiteren stellt die vorliegende Erfindung auch eine Nukleinsäure bereit, die für ein wie oben beschriebenes Peptid, Polypeptid oder Protein kodiert. Eine Nukleinsäure, die für ein Kapsid-Protein kodiert, welches ein wie oben beschriebenes, erfindungsgemäßes Peptid, Polypeptid oder Protein umfasst, wird ebenfalls bereitgestellt. Vorzugsweise umfasst die für das Kapsid-Protein kodierende Nukleinsäure die Nukleotidsequenz von SEQ ID NO:11 oder eine davon abgeleitete Nukleotidsequenz mit mindestens 80% Sequenzidentität. Ein Plasmid, das eine solche Nukleinsäure umfasst, wird ebenfalls bereitgestellt.

In einem noch weiteren Aspekt betrifft die Erfindung einen rekombinanten, d.h. einen mittels gentechnischer Verfahren hergestellten, viralen Vektor, der ein Kapsid und mindestens ein darin verpacktes Transgen umfasst, wobei das Kapsid mindestens ein Kapsid-Protein umfasst, welches die Peptidsequenz gemäß SEQ ID NO:1 oder einer Variante davon oder gemäß SEQ ID NO:2-6 oder einer Variante davon umfasst. Bei dem rekombinanten viralen Vektor kann es sich um einen rekombinanten AAV-Vektor handeln, z.B. um einen solchen vom Serotyp 2, 4, 6, 8 und 9. AAV-Vektoren vom Serotyp 2 sind besonders bevorzugt.

Die unterschiedlichen AAV-Serotypen unterscheiden sich im Wesentlichen anhand ihres natürlichen Tropismus. So bindet Wildtyp-AAV2 stärker an alveolare Zellen, während AAV5, AAV6 und AAV9 vorwiegend Epithelzellen infizieren. Der Fachmann kann diese natürlichen Unterschiede hinsichtlich der Spezifität der Zellen nutzen, um die durch die erfindungsgemäßen Peptide vermittelte Spezifität für bestimmte Zellen oder Gewebe weiter zu verstärken. Auf der Nukleinsäureebene sind die verschiedenen AAV-Serotypen stark homolog. So sind z.B. die Serotypen AAV1, AAV2, AAV3 und AAV6 auf der Nukleinsäureebene zu 82% identisch [25].

Das Kapsid der erfindungsgemäßen viralen Vektoren umfasst vorzugsweise ein oder mehrere Transgene. Als Transgen wird vorliegend ein Gen bezeichnet, das mittels gentechnischer Verfahren in das Genom des Vektors eingebracht wurde. Bei dem Transgen (oder den Transgenen) kann es sich um DNA oder RNA handeln. Vorzugsweise handelt es sich um einzelsträngige DNA (ssDNA) oder doppelsträngige DNA (dsDNA), wie beispielsweise genomische DNA oder cDNA. Vorzugsweise handelt es sich bei dem Transgen, welches mit Hilfe des erfindungsgemäßen rekombinanten viralen Vektors transportiert werden soll, um ein humanes Gen. Geeignete Transgene sind z.B. therapeutische Gene, die ein im Patienten dysfunktionales Gen ersetzen sollen. Es kann sich ferner um ein Gen handeln, das in dem entsprechenden Zielgewebe nicht oder nur in unzureichendem Umfang exprimiert wird. In einer bevorzugten Ausführungsform handelt es sich bei dem Transgen, welches vom viralen Vektor der Erfindung exprimiert wird, um ein.

Die erfindungsgemäßen Vektoren können u.a. zur Behandlung der multiplen Sklerose (MS) eingesetzt werden. In diesem Fall kann es sich bei dem Transgen z.B. um ein Gen handeln, das für ein Membran- oder Tight-Junction-Protein kodiert, wie z.B. für ein Claudin und Occludin. Verzugsweise handelt es sich um ein Gen aus der Familie der Claudine handeln, z.B. um das für Claudinl kodierende Gen. Die Überexpression eines solchen Gens könnte hilfreich sein, um die durch die Krankheit geschädigte Blut-Hirn-Schranke "abzudichten".

Ferner kann zur Behandlung der MS auch ein Gen eingesetzt werden, das für einen Chemokin-Antagonisten kodiert. So kann z.B. eine negativ-dominante Mutante des Chemokins CCL2 (aka MCP1) exprimiert werden, die als "CCL2-7ND" bezeichnet wird. CCL2 sorgt dafür, dass Immunzellen zu einem Entzündungsherd gelockt werden, was im Fall der MS zum entzündlichen Abbau der neuronalen Myelinscheide führt. Die dominant-negative Variante "CCL2-7ND" hingegen bildet Dimere und blockiert den Rezeptor CCR2, wodurch die Signalkaskade unterbunden wird und die Immunzellen nicht mehr zu den Neuronen gelockt werden.

Zur Behandlung der Alzheimer-Krankheit kann z.B. das kodierende Gen für Neuraminidasel (NEU1), Neprilysin oder Cholesterol 24-Hydroxylase überexprimiert werden, was zur Abreicherung von Amyloid ß-Plaques führen und damit zu einer Verbesserung des Krankheitszustands beitragen kann.

Die Parkinson Krankheit kann durch Vektor-vermittelte Überexpression z.B. des neurotrophischen Faktors der Gliazellen (GDNF), der aromatischen L-Aminosäure-Decarboxylase, der Tyrosynhydroxylase oder der GTP-Cyclohydrolase I behandelt werden, wobei ein positiver Einfluss auf das dopaminergische System zu erwarten wäre. Eine Behandlung der Spinalen Muskelatrophie kann z.B. durch Expression des Proteins SMN (Survival of Motor Neuron) erfolgen.

Für die Therapie einer lysosomalen Speicherkrankheit wäre die Expression einer Glucuronidase, z.B. der ß-Glucuronidase, als Transgen mit Hilfe des erfindungsgemäßen Vektors erfolgsversprechend.

In einer weiteren Ausführungsform kodiert das Transgen für ein radioprotektives Protein, wie z.B. für ein radioprotektives Enzym. Eine wesentliche Limitation beim Einsatz der Radiotherapie im Rahmen der Behandlung von Krebs ist die strahleninduzierte Schädigung des Normalgewebes, was häufig eine Verringerung der Strahlendosis, eine Unterbrechung des Therapieregimes, oder sogar einen völligen Verzicht auf diese Therapieform nötig macht. Das Gehirn ist ein besonders strahlenempfindliches Organ, weshalb Primärtumoren dort oft nur eingeschränkt und diffuses Tumorwachstum meistens gar nicht radiotherapeutisch behandelt werden kann. Mit Hilfe der Vektoren der vorliegenden Erfindung kann das gesunde Gewebe, welches das maligne Gewebe umgibt, durch zielgerichtete Expression radioprotektiver Proteine geschützt werden. In einer bevorzugten Ausführungsform handelt es sich bei dem Transgen, welches in das gesunde Gehirngewebe eingeschleust wird, um eine Mangan-Superoxiddismutase (MnSOD), die die Konversion von Superoxidanionen, einem der wesentliche Faktoren der strahleninduzierten Toxizität, zu Wasserstoffperoxid katalysiert. In einer weiteren hier vorgeschlagenen Ausführungsform handelt es sich um die Kinase-Domäne des Ataxia telangiectasia mutant (ATM)-Gens, das zur Reparatur der durch Bestrahlung entstandenen DNA-Schäden beiträgt. In einer weiteren bevorzugten Ausführungsform werden beide Gene mittels der vorliegend beschriebenen Vektoren in den zu behandelnden Patienten eingebracht.

Die viralen Vektoren der vorliegenden Erfindung eignen sich insbesondere zur Verwendung in einem Verfahren zur therapeutischen Behandlung von Erkrankungen des Gehirns und/oder Rückenmarks. Erkrankungen des Gehirns bzw. des Rückenmarks im Sinne der Erfindung umfassen genetisch verursachte Leukodystrophien, wie Adrenoleukodystrophie, Morbus Canavan, Morbus Krabbe, metachromatische Leukodystrophie, Pelizaeus-Merzbacher-Krankheit und Morbus Alexander; neurodegenerative Erkrankungen, wie amyotrophe Lateralsklerose, Alzheimer, Parkinson, Chorea Huntington und Morbus Pick; chronisch-entzündliche Erkrankungen des Zentralnervensystems, wie Multiple Sklerose und Guillain-Barre-Syndrom; und Lysosomen-Speichererkrankungen, wie Ceroid-Lipofuszinose und Morbus Fabry. In einer bevorzugten Ausführungsform werden die viralen Vektoren der vorliegenden Erfindung zur therapeutischen Behandlung von neurodegenerative Erkrankungen, wie Alzheimer, Parkinson oder Chorea Huntington eingesetzt.

In einer noch weiteren Ausführungsform kodiert das Transgen für ein Antitumormittel, wie z.B. ein Tumorsupressor-Protein, oder einen Immunmodulator, wie z.B. ein Zytokin (z.B. Interleukin 1 bis 4, Gamma-Interferon, p53), das selektiv zu einem Gehirntumor oder einen Tumor des Rückenmarks des Patienten transportiert werden soll.

Die Vektoren können ferner auch dazu verwendet werden, um Antisense-RNA, Ribozyme, oder Ähnliches in Zellen und Gewebe des Gehirns bzw. Rückenmarks zu transportieren. Ferner können die erfindungsgemäßen Vektoren auch Transgene umfassen, die für sekretorische Proteine kodieren, die in das Lumen der Mikrovaskulatur des Gehirns oder des Rückenmarks freigesetzt werden sollen. Solche sekretorischen Proteine können beispielsweise entzündungshemmend wirken.

Wie vorliegend verwendet, bezeichnet der Begriff "Subjekt" alle menschlichen oder tierischen Organismen, die durch AAV-Vektoren infiziert werden können. Vorzugsweise handelt es sich bei dem zu behandelnden Subjekt um ein Säugetier, wie z.B. um einen Menschen, einen Primaten, eine Maus oder eine Ratte. In einer bevorzugten Ausführungsform ist das zu behandelnde Subjekt ein Mensch. Nach Transfektion in das Subjekt sorgt der Vektor für die ortsspezifische Expression des Transgens in den Zellen des Gehirns bzw. des Rückenmarks.

Das Transgen kann in dem viralen Vektor in Form einer Expressionskassette vorliegen, die neben der zu exprimierenden Sequenz des Transgens weitere für die Expression erforderliche Elemente umfasst, wie z.B. einen geeigneten Promoter, der nach Infektion der entsprechenden Zellen die Expression des Transgens steuert. Geeignete Promotoren umfassen neben den AAV-Promotoren z.B. den Cytomegalovirus (CMV)-Promoter, den Chicken-Beta-Actin/Cytomegalovirus-Hybridpromoter (CAG), einen Endothelzellen-spezifischen Promoter, wie z.B. den VE-Catherin Promoter, sowie Steroid-Promotoren und Metallothionein-Promotoren. In einer besonders bevorzugten Ausführungsform ist der in den erfindungsgemäßen Vektoren verwendete Promoter ein CAG-Promoter. In einer besonders bevorzugten Ausführungsform umfasst das erfindungsgemäße Transgen einen hirnspezifischen Promoter, der in funktionaler Verbindung mit dem zu exprimierenden Transgen verbunden ist. Auf diese Weise kann die Spezifität der erfindungsgemäßen Vektoren für das Gehirn weiter erhöht werden. Wie hierin verwendet, ist ein hirnspezifischer Promoter ein Promoter, dessen Aktivität in Hirngewebe mindestens 2-fach, 5-fach, 10-fach, 20-fach, 50-fach oder 100-fach höher ist als in einer Zelle, die keine Gehirnzelle ist. Vorzugsweise ist dieser Promoter ein humaner Promoter. Die Expressionskassette kann auch ein Enhancer-Element zur Erhöhung des Expressionslevels des zu exprimierenden exogenen Proteins enthalten. Ferner kann die Expressionskassette Polyadenylierungssequenzen, wie z.B. die SV40-Polyadenylierungssequenzen oder die Polyadenylierungssequenzen des bovinen Wachstumshormons umfassen.

Die erfindungsgemäßen viralen Vektoren können als Bestandteil eines ihrer Kapsid-Proteine vorzugsweise eine Peptidsequenz gemäß einer der in den SEQ ID NO:1-5 umfassen. Alternativ können auch Varianten der Aminosäuresequenz von SEQ ID NO:1-5 verwendet werden, die sich von der Aminosäuresequenz von SEQ ID NO:1-5 durch Modifikation von einer Aminosäure unterscheiden. Bei der Modifikation kann es sich um einen Austausch, eine Deletion oder eine Insertion von Aminosäuren handeln, solang die Variante die Fähigkeit beibehält, als Teil des Kapsids die spezifische Bindung des Vektors an Rezeptor-Strukturen von Zellen des Gehirns und/oder Rückenmarks zu vermitteln. Die Erfindung erstreckt sich somit z.B. auf Varianten der Sequenz von SEQ ID NO:1-5, bei denen die C- oder N-terminale Aminosäure verändert wurde. Diese Varianten weisen eine Sequenzidentität von mehr als 85% zu der in SEQ ID NO:1-5 gezeigten Aminosäuresequenz auf, wenn die Sequenzen mit den Programmen GAP oder BESTFIT miteinander verglichen werden. Diese Computerprogramme zur Bestimmung der Aminosäuresequenzidentität sind im Stand der Technik hinreichend bekannt.

Die Variante der Sequenz gemäß SEQ ID NO:1-5 kann auf einer Substitution einer Aminosäure beruhen, d.h. eine Aminosäure kann gegen eine andere Aminosäure ausgetauscht sein. Vorzugsweise ist die Substitution, durch die sich die Varianten von einer der in SEQ ID NO:1-5 dargestellten Aminosäuresequenzen unterscheidet, eine konservative Substitution, d.h. eine Substitution einer Aminosäure durch eine Aminosäure ähnlicher Polarität, die dem Peptid ähnliche funktionelle Eigenschaften verleiht. Vorzugsweise stammt die ausgetauschte Aminosäure aus der gleichen Gruppe von Aminosäuren wie die ersetzende Aminosäure. Beispielsweise kann ein hydrophober Rest durch einen anderen hydrophoben Rest ersetzt werden oder ein polarer Rest durch einen anderen polaren Rest. Funktionell ähnliche Aminosäuren, die im Rahmen einer konservativen Substitution gegeneinander ausgetauscht werden können, umfassen z.B. nicht-polare Aminosäuren wie z.B. Glycin, Valin, Alanin, Isoleucin, Leucin, Methionin, Prolin, Phenylalanin, und Tryptophan. Beispiele von ungeladenen polaren Aminosäuren umfassen Serin, Threonin, Glutamin, Asparagin, Tyrosin, und Cystein. Beispiele von geladenen polaren (sauren) Aminosäuren umfassen Histidin, Arginin und Lysin. Beispiele von geladenen polaren (basischen) Aminosäuren umfassen Asparaginsäure und Glutaminsäure.

Als Varianten der in SEQ ID NO:1-5 gezeigten Aminosäuresequenzen gelten auch solche Aminosäuresequenzen, bei denen eine Aminosäure eingefügt wurde. Solche Insertionen können erfolgen, so lange die resultierende Variante ihre Fähigkeit beibehält, spezifisch an Zellen des Gehirns und/oder des Rückenmarks zu binden. Ferner gelten vorliegend auch solche Proteine als Varianten der in SEQ ID NO:1-5 gezeigten Aminosäuresequenzen, bei denen eine der beiden N-terminalen Aminosäuren des Peptids fehlt. Voraussetzung ist wiederum, dass die entsprechend deletierte Variante spezifisch an Zellen des Gehirns und/oder des Rückenmarks bindet.

Ebenfalls von der Erfindung umfasst sind spezifische Varianten der in SEQ ID NO:1-5 gezeigten Aminosäuresequenzen, die an einer Aminosäure strukturell verändert wurden, wie beispielsweise durch Einführen einer modifizierten Aminosäure. Bei diesen modifizierten Aminosäuren kann es sich erfindungsgemäß um Aminosäuren handeln, die durch Biotinylierung, Phosphorylierung, Glykosylierung, Acetylierung, Verzweigung und/oder Zyklisierung verändert wurden.

Virale Vektoren, deren Kapsid eine der erfindungsgemäßen Peptidsequenzen oder eine wie oben definierte Variante davon umfassen, binden spezifisch Zellen des Gehirns und/oder des Rückenmarks. Wie vorliegend verwendet bedeutet eine "spezifische" Bindung der erfindungsgemäßen Vektoren, dass sich die Vektoren nach systemischer Verabreichung vorwiegend an oder in den Zellen des Gehirns und/oder des Rückenmarks anreichern. Dies bedeutet, dass sich mehr als 50% der ursprünglich verabreichten Vektorgenome im Bereich der Zellen des Gehirns und/oder des Rückenmarks anreichern, während sich weniger als 50% in oder im Bereich von anderen Zellen oder Geweben anreichern (wie z.B. in der Milz oder Leber). Es ist bevorzugt, dass sich mehr als 60%, 70%, 80%, 90%, 95%, oder sogar mehr als 99% der ursprünglich verabreichten Vektorgenome in oder im Bereich der Zellen des Gehirns und/oder des Rückenmarks anreichern. Eine spezifische Bindung der Vektoren kann auch über die Expression des Transgens ermittelt werden. Bei viralen Vektoren, die spezifisch Zellen des Gehirns und/oder des Rückenmarks binden, erfolgt mehr als 50% der gesamten Expression des Transgens in oder im Bereich des Gehirns und/oder des Rückenmarks, während weniger als 50% der Expression in oder im Bereich von anderen Geweben beobachtet werden kann. Es ist allerdings bevorzugt, dass mehr als 60%, 70%, 80%, 90%, 95%, oder sogar mehr als 99% der insgesamt gemessenen Expression des Transgens in oder im Bereich des Gehirns und/oder des Rückenmarks erfolgt.

Der Fachmann wird problemlos in der Lage sein, die spezifische Bindung der erfindungsgemäßen Vektoren an Zellen des Gehirns bzw. des Rückenmarks und die Expression des mit Hilfe der Vektoren eingeschleusten Transgens zu bestimmten. Zu diesem Zweck geeignete Verfahren zur Messung der Spezifität von Transduktion und Expression sind in den nachstehenden Beispielen gezeigt.

Es ist darüber hinaus erfindungsgemäß bevorzugt, dass Vektoren, deren Kapside Varianten der in SEQ ID NO:1-5 gezeigten Aminosäuresequenz enthalten, mindestens etwa 50% der Bindungsaktivität eines entsprechenden viralen Vektors aufweisen, dessen Kapsid die in SEQ ID NO:1-5 gezeigte Aminosäuresequenz aufweist. Noch stärker bevorzugt ist es, dass die Varianten etwa 60%, 70%, 80%, 90%, 95%, 96%, 97%, 98% oder 99% der Bindungsaktivität eines entsprechenden viralen Vektors aufweisen, dessen Kapsid die in SEQ ID NO:1-5 gezeigte Aminosäuresequenz aufweist. Die Bindungsaktivität der Vektoren kann mit Hilfe von in vitro-Assays gemessen werden, wie sie in den vorliegenden Beispielen beschrieben werden.

Vorzugsweise ist die Bindungsaktivität der erfindungsgemäßen Vektoren, wie sie durch Verteilung der Vektorgenome oder Expression des Transgens bestimmt werden kann, für Zellen des Gehirns und/oder des Rückenmarks mindestens 2-fach, 5-fach, 10-fach, 20-fach, 50-fach, 75-fach, 100-fach, 150-fach, 200-fach, 250-fach, 500-fach, 600-fach, 700-fach, 800-fach, 900-fach, 1000-fach, 2000-fach, 5000-fach, oder 10000-fach höher als die Bindungsaktivität für eine Kontroll-Zelle, die keine Zelle des Gehirns oder Rückenmarks ist, Z.B. eine Milz- oder Leber-Zelle).

Die Erfindung betrifft auch eine Zelle, die ein erfindungsgemäßes Peptid, Polypeptid oder Protein, eine dafür kodierende Nukleinsäure, ein Plasmid, welches eine solche Nukleinsäure umfasst, oder einen wie oben beschriebenen rekombinanten AAV-Vektor enthält. Es handelt sich vorzugsweise um eine humane Zelle oder Zelllinie.

In einer Ausführungsform wurde eine Zelle z.B. durch ein Biopsieverfahren aus einem humanen Subjekt gewonnen und anschließend mit dem viralen Vektor in einem *ex vivo* Verfahren transfiziert. Die Zelle kann anschließend dem Subjekt reimplantiert oder diesem auf andere Weise wieder zugeführt werden, z.B. durch Transplantation oder Infusion. Die Wahrscheinlichkeit einer Abstoßung von transplantierten Zellen ist geringer, wenn das Subjekt, aus dem die Zelle gewonnen wurde, genetisch dem Subjekt ähnelt, an das die Zelle verabreicht wird. Vorzugsweise handelt es sich daher bei dem Subjekt, dem die transfizierten Zellen zugeführt werden, um dasselbe Subjekt, aus dem zuvor die Zellen gewonnen wurden. Die Zelle ist vorzugsweise eine humane Zelle des Gehirns oder Rückenmarks, insbesondere eine neuronale Zelle, eine Endothelzelle eines Blutgefäßes. Die die zu transfizierende Zelle kann auch eine Stammzelle, wie z.B. eine adulte humane Stammzelle, sein. Es ist erfindungsgemäß besonders bevorzugt, dass es sich bei den zu transfizierenden Zellen um autologen Zellen handelt, die mit dem erfindungsgemäßen viralen Vektor, z.B. dem beschriebenen rekombinanten AAV2-Vektor, *ex vivo* transfiziert wurde. Die Zellen werden vorzugsweise in einem Verfahren zur Behandlung einer Funktionsstörung oder Erkrankung des Gehirns und/oder des Rückenmarks in einem Subjekt verwendet.

In einem weiteren Aspekt betrifft die Erfindung ein Verfahren zur Herstellung eines AAV-Vektors, bei dem ein Plasmid verwendet wird, dass für ein Kapsid-Protein kodiert, welches die Aminosäuresequenz von SEQ ID NO:1 oder einer Variante davon umfasst. Alternativ kann der Vektor auch eine Aminosäuresequenz gemäß SEQ ID NO:6 umfassen, insbesondere eine der Aminosäuresequenzen gemäß SEQ ID NO:2-5 oder eine Variante davon. Das grundsätzliche Verfahren der Herstellung von rekombinanten AAV-Vektoren, die ein zu exprimierendes Transgen umfassen, ist im Stand der Technik hinreichend beschrieben [28]. HEK293-T Zellen werden mit drei Plasmiden transfiziert. Ein erstes Plasmid enthält das die cap- und rep-Regionen des AAV-Genoms, wobei jedoch die natürlich vorkommenden Inverted Repeats (ITRs) fehlen. Die cap-Region dieses Plasmids enthält ein Gen, das für mindestens ein modifiziertes Kapsid-Protein kodiert, d.h. ein Gen, welches die erfindungsgemäße Peptidsequenz umfasst. Ein zweites Plasmid umfasst eine Transgen-Expressionskassette, die von den entsprechenden ITRs flankiert wird, die das Verpackungssignal darstellen. Die Expressionskassette wird daher im Zuge des Zusammenbaus der Viruspartikel in das Kapsid verpackt. Bei dem dritten Plasmid handelt es sich um ein adenovirales Helferplasmid, auf dem die Helferproteine E1A, E1B, E2A, E4-orf6, VA kodiert sind, die für die AAV-Replikation in den HEK 293-T-Zellen erforderlich sind. Alternativ ist auch möglich, die erfindungsgemäßen AVV-Vektoren in Insektenzellen zu erzeugen. Ein entsprechendes Protokoll ist beispielhaft im unten aufgeführten Beispiel 6 aufgeführt.

Es hat sich überraschend gezeigt, dass die Herstellung der rekombinanten Vektoren deren Spezifität beeinflussen kann. So wurde im Rahmen der vorliegenden Erfindung beobachtet, dass Vektoren, die mit Hilfe des Protokolls von Beispiel 2 in HEK293T-Zellen hergestellt wurden, vorwiegend Endothelzellen der Blutgefäße von Gehirn und Rückenmark transfizierten und nur zu einem geringeren Teil Neuronen des Gehirns oder des Rückenmarks. Anderseits transfizierten die rekombinanten Vektoren, die mit Hilfe des Protokolls von Beispiel 6 in Sf9-Zellen hergestellt wurden, vorwiegend Neuronen und im wesentlich geringeren Umfang Endothelzellen. Diese Beobachtung kann entsprechend genutzt werden, um die Spezifität der Transfektion weiter zu erhöhen.

Bedingungen, welche die Anreicherung und Aufreinigung der erfindungsgemäßen rekombinanten Vektoren erlauben, sind im Stand der Technik bekannt. Die erfindungsgemäßen Vektoren können beispielsweise durch Gelfiltrationsverfahren, z.B. unter Verwendung einer Sepharosematrix, entsalzt und durch anschließende Filtration aufgereinigt werden. Andere Aufreinigungsverfahren können ferner eine Cäsiumchlorid- oder Iodixanol-Gradienten-Ultrazentrifugation umfassen. Die Aufreinigung reduziert potentiell abträgliche Affekte in dem Subjekt, an das die adeno-assoziierten viralen Vektoren verabreicht werden. Der verabreichte Virus ist im Wesentlichen frei von Wildtyp- und replikationskompetenten Virus. Die Reinheit des Virus kann durch geeignete Verfahren, wie PCR-Amplifikation, überprüft werden.

Die Erfindung stellt in einem weiteren Aspekt eine pharmazeutische Zusammensetzung bereit, die einen viralen Vektor der vorliegenden Erfindung, insbesondere einen AAV-Vektor, umfasst. Der virale Vektor wird dabei in einer therapeutisch wirksamen Menge an den Patienten verabreicht, d.h. in einer Menge, die ausreicht, um mindestens ein Symptom der zu behandelnden Funktionsstörung oder Erkrankung des Gehirns und/oder des Rückenmarks in einem erheblichen Maße zu verbessern oder die Progression der Erkrankung zu unterbinden. Eine therapeutisch wirksame Menge des erfindungsgemäßen Vektors ruft eine positive Veränderung in einem der besagten Symptom hervor, d.h. eine Veränderung die den Phänotyp des betroffenen Subjekts an den Phänotyp eines gesunden Subjekts, das nicht unter einer Erkrankung des Gehirns und/oder des Rückenmarks leidet, annähert.

In einer bevorzugten Ausführungsform der Erfindung erfolgt die Verabreichung des viralen Vektors in einer Menge, die zu einer vollständigen oder im Wesentlichen vollständigen Heilung der Funktionsstörung oder Erkrankung des Gehirns und/oder des Rückenmarks führt. Die pharmazeutische Zusammensetzung wird demgemäß eine therapeutisch wirksame Dosis des erfindungsgemäßen Vektors enthalten. Eine therapeutisch wirksame Dosis wird in der Regel für das Subjekt, welches sich der Behandlung unterzieht, nicht toxisch sein.

Die genaue Menge an viralen Vektor, die verabreicht werden muss, um einen therapeutischen Effekt zu erzielen, hängt von mehreren Parametern ab. Faktoren, die relevant für die Menge des zu verabreichenden viralen Vektors sind, umfassen z.B. die Art der Verabreichung des viralen Vektors, die Art und Schwere der Erkrankung, die Krankheitsgeschichte des zu behandelnden Patienten sowie Alter, Gewicht, Größe und Gesundheitszustand des zu behandelnden Patienten. Ferner sind auch das Expressionslevel des Transgens, das benötigt wird um einen therapeutischen Effekt zu erzielen, die Immunantwort des Patienten, sowie die Stabilität des Genproduktes für die zu verabreichende Menge relevant. Eine therapeutisch wirksame Menge des viralen Vektors kann von einem Fachmann ohne Weiteres auf Basis des allgemeinen Fachwissens und der vorliegenden Offenbarung bestimmt werden.

Der virale Vektor wird vorzugsweise in einer Menge verabreicht, die einer Virusdosis im Bereich von 1,0 x 10¹⁰ bis 1,0 x 10¹⁴ vg/kg (Virusgenome pro kg Körpergewicht) entspricht, wobei ein Bereich von 1,0 x 10¹¹ bis 1,0 x 10¹³ vg/kg stärker bevorzugt ist, und ein Bereich von 5,0 x 10¹¹ bis 5,0 x 10¹² vg/kg stärker bevorzugt ist, und ein Bereich von 1,0 x 10¹² bis 5,0 x 10¹² noch stärker bevorzugt ist. Eine Virusdosis von etwa 2,5 x 10¹² vg/kg ist am stärksten bevorzugt. Die zu verabreichende Menge des viralen Vektors, z.B. des erfindungsgemäßen AAV2-Vektors, kann abhängig von der Stärke der Expression des einen oder der mehreren Transgene angepasst werden.

Der virale Vektor der vorliegenden Erfindung, wie z.B. der erfindungsgemäß bevorzugte AVV2-Vektor, kann für verschiedene Arten der Verabreichung formuliert werden, z.B. für die orale Verabreichung als Kapsel, als Flüssigkeit oder Ähnliches. Es ist jedoch bevorzugt, dass der virale Vektor parenteral, vorzugsweise mittels intravenöser Injektion oder intravenöser Infusion verabreicht wird. Die Verabreichung kann beispielsweise mittels intravenöser Infusion, z.B. innerhalb von 60 Minuten, innerhalb von 30 Minuten, oder innerhalb von 15 Minuten erfolgen. Es ist ferner bevorzugt, dass der virale Vektor während einer Operation lokal mittels Injektion in das Gehirn und/oder das Rückenmark verabreicht wird. Zusammensetzungen, die für die Verabreichung mittels Injektion und/oder Infusion geeignet sind, umfassen in der Regel Lösungen und Dispersionen sowie Pulver, aus denen entsprechende Lösungen und Dispersionen hergestellt werden können. Derartige Zusammensetzungen werden den viralen Vektor und mindestens einen geeigneten pharmazeutisch akzeptablen Träger enthalten. Geeignete pharmazeutisch akzeptable Träger für die intravenöse Verabreichung umfassen bakteriostatisches Wasser, Ringer-Lösung, physiologische Salzlösung, Phosphat-gepufferte Salzlösung (PBS) und Cremophor EL™. Sterile Zusammensetzungen für die Injektion und/oder Infusion können hergestellt werden, indem der viralen Vektor in der erforderlichen Menge in einem geeigneten Träger eingebracht und anschließend mittels eines Filters sterilfiltriert wird. Zusammensetzungen für die Verabreichung mittels Injektion oder Infusion sollten nach ihrer Herstellung unter Lagerungsbedingungen über einen längeren Zeitraum stabil bleiben. Die Zusammensetzungen können zu diesem Zweck ein Konservierungsmittel enthalten. Geeignete Konservierungsmittel umfassen Chlorbutanol, Phenol, Ascorbinsäure und Thiomersal. Die Herstellung von entsprechenden Darreichungsformen sowie geeignete Hilfsstoffe sind beispielsweise in "Remington: The Science and Practice of Pharmacy", Lippincott Williams & Wilkins; 21. Auflage (2005) beschrieben.

In noch einem weiteren Aspekt betrifft die Erfindung ein Verfahren zur therapeutischen Behandlung einer Funktionsstörung oder einer Erkrankung des Gehirns und/oder des Rückenmarks, bei dem man einen erfindungsgemäßen viralen Vektor, vorzugsweise einen AAV-Vektor, wie er oben beschrieben ist, an ein Subjekt verabreicht. Der Vektor umfasst ein Kapsid, das mindestens ein Kapsid-Protein umfasst, welches die Peptidsequenz gemäß SEQ ID NO:1 oder einer Variante davon enthält. Alternativ kann der Vektor auch eine Aminosäuresequenz gemäß SEQ ID NO:6 umfassen, insbesondere eine der Aminosäuresequenzen gemäß SEQ ID NO:2-5 oder eine Variante davon. Der virale Vektor umfasst ferner ein Transgen, z.B. ein therapeutisches Gen, das für die Behandlung der Funktionsstörung oder Erkrankung des Gehirns und/oder des Rückenmarks nützlich ist. Nach Verabreichung an das zu behandelnde Subjekt, vorzugsweise durch systemische Gabe, wie z.B. intravenöse Injektion oder Infusion, sorgt der Vektor für die spezifische Expression des Gens in den Zellen des Gehirns und/oder des Rückenmarks.

### KURZE BESCHREIBUNG DER FIGUREN

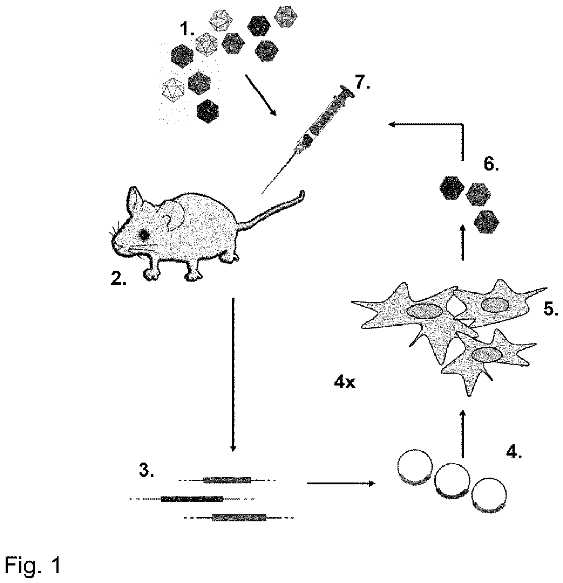

**Figur 1** zeigt das im Rahmen der Erfindung angewendete *in vivo* Selektionsverfahren der AAV-Peptidbank.
   1.: Randomisierte AAV Peptidbibliothek mit ∼1x10⁸ unterschiedlichen Kapsidvarianten;
   2.: Entnahme des Zielorgans, 8 Tage nach Injektion;
   3.: DNS Isolierung und Amplifikation der viralen DNS-Fragmente per Real-Time-PCR;
   4.: Klonierung in Peptidbibliotheks-Plasmide für Sequenzierung und Produktion einer sekundären Peptidbibliothek;
   5.: Co-Transfektion von HEK293T Zellen, Produktion einer sekundären Peptidbibliothek;
   6.: sekundäre AAV Peptidbibliothek für weitere Selektionsrunden. Beinhaltet vorselektierte Kapsidvarianten;
   7.: intravenöse Injektion der Peptidbibliothek in die Maus.

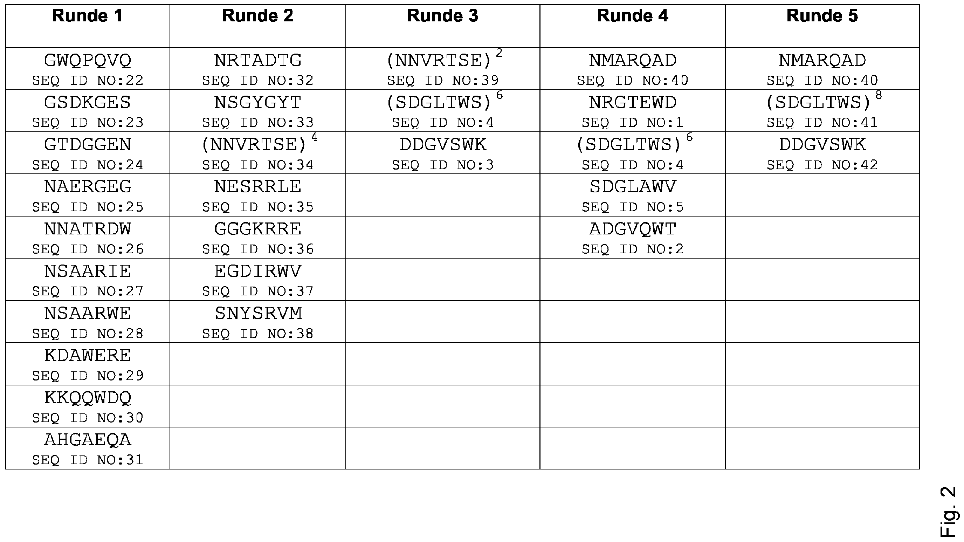

**Figur 2** zeigt die Sequenzen der mittels des Selektionsverfahrens identifizierten, für das Gehirn spezifischen Peptide. Das Nach Peptid mit der in SEQ ID NO:1 gezeigten Sequenz wurde in der vierten Selektionsrunde identifiziert.

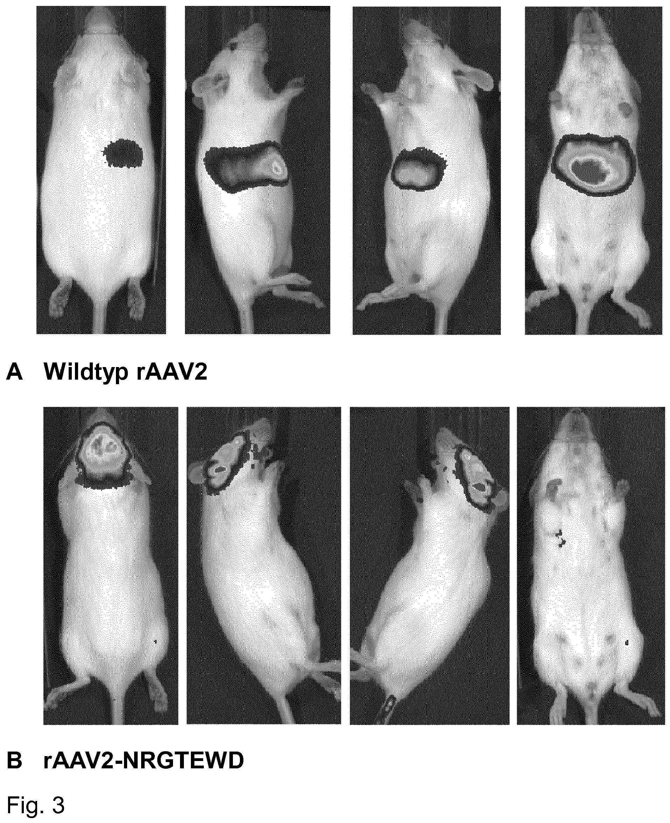

**Figur 3** zeigt die Genexpression nach intravenöser Injektion von rekombinanten AAV-Vektoren in der lebenden Maus. Die Luziferaseexpression wurde 14 Tage nach systemischer Injektion von 5x10¹⁰ Vektorgenomen mit einem IVIS® 200 Imaging Imaging System gemessen. A: Vektoren auf Basis des unmodifizierten AAV2-Wildtyp-kapsids zeigen Genexpression hauptsächlich in der Leber. Es kommt zu keine messbaren Genexpression im Gehirn. B: Der erfindungsgemäße rekombinante AAV-Vektor rAAV2-NRGTEWD hingegen vermittelt eine starke und spezifische Genexpression im Gehirn.

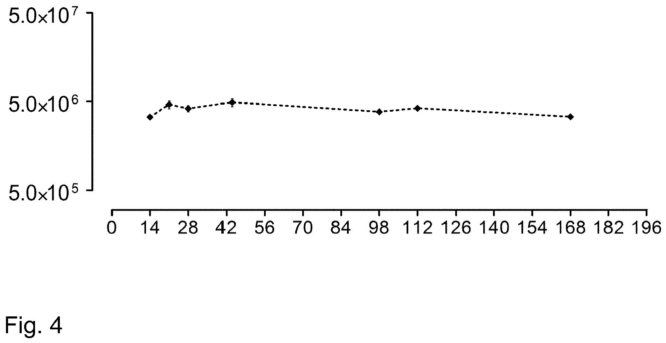

**Figur 4** zeigt eine Langzeit-Expressionsanalyse in der Maus nach systemischer Gabe des rekombinanten AAV-Vektors rAAV2-NRGTEWD. Wiederholte Messungen mittels IVIS® 200 Imaging System zeigen eine stabile Genexpression im Gehirn über einen Zeitraum von 168 Tagen (n=2).

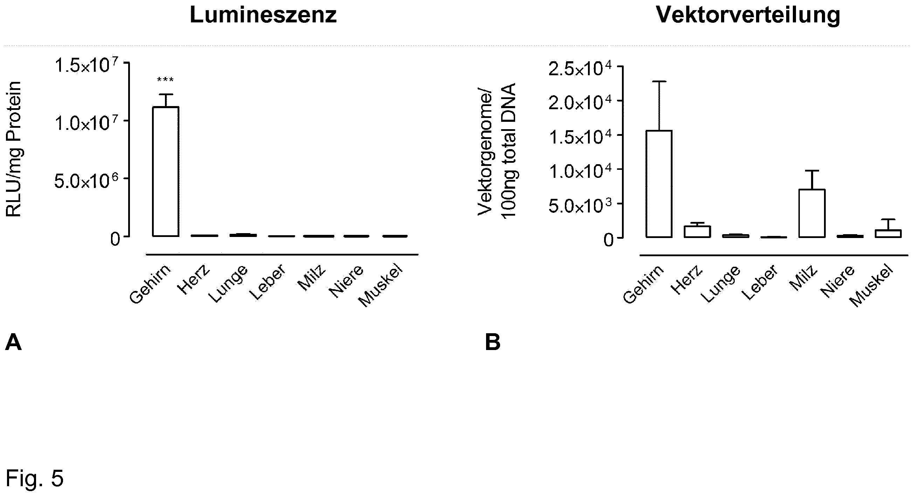

**Figur 5** zeigt die Ergebnisse der Bestimmung von Lumineszenz und Vektorverteilung 14 Tage nach intravenöser Injektion von 5x10¹⁰ Vektorgenomen des rekombinanten AAV-Vektors rAAV2-NRGTEWD. A: Die Messung der Genexpression als Lumineszenz in den Lysaten verschiedener Organe der Maus zeigt hohe Spezifität des Vektors für das Gehirn. B: Die Messung der Vektorgenom-Verteilung in den Lysaten zeigt eine deutliche Anreicherung des Vektors im Gehirn. Mittelwerte + Standartabweichung. Die Statistik wurde mittels One-Way ANOVA berechnet. P-Werte sind p<0,05=*; p<0,01=**; p<0,001=*** für n=3.

### BEISPIELE

Alle Daten wurden als Mittelwerte + Standardabweichung (SD) bestimmt. Die statistische Analyse wurde unter Verwendung des GraphPad Prism Programms 3.0 (GraphPad Software, San Diego, USA) durchgeführt. Die Daten wurden durch Einweg-ANOVA, gefolgt von multiplen Vergleichstests nach Bonferoni analysiert. P-Werte >0,05 wurden als signifikant erachtet.

### Beispiel 1: Selektion von AAV2-Peptidbanken

Um gewebespezifische AAv2-Kapside zu selektieren, wurde eine randomisierte AAV-Peptidbank hergestellt und über fünf Runden selektiert. Eine zufallsbasierte X₇-AAV-Peptidbank mit einer theoretischen Diversität von 1x10⁸ einzeln vorkommenden Klonen wurde unter Verwendung eines zweistufigen Protokolls hergestellt, wie es zuvor beschrieben wurde [26-27]. Es wurde zunächst ein degeneriertes Oligonukleotid hergestellt, das für sieben zufällig angeordnete Aminosäuren an der Nukleotidposition 3967 im AAV-Genom kodiert, was der Aminosäureposition R588 in VP1 entspricht. Das Oligonukleotid hatte die Sequenz: 5' - CAGTCGGCCAGAGAGGC (NNK)₇GCCCAGGCGGCTGACGAG - 3' (SEQ ID NO:12) . Der zweite Strang wurde unter Verwendung einer Sequenase (Amersham, Freiburg, Deutschland) und des Primers mit der Sequenz 5'-CTCGTCAGCCGCCTGG-3' (SEQ ID NO:13) hergestellt. Das doppelsträngige Insert wurde mit BglI geschnitten, mit dem QIAquick Nucleotide Removal Kit (Qiagen, Hilden, Deutschland) aufgereinigt und in das mit SfiI verdaute Bibliothek-Plasmid pMT187-0-3 ligiert [26]. Die Diversität der Plasmid-Bibliothek wurde anhand der Anzahl von Klonen bestimmt, die ausgehend von einem repräsentativen Aliquot von transformierten, elektrokompetenten DH5α-Bakterien auf Agar wuchsen, der 150 mg/ml Ampicillin enthielt. Bibliothek-Plasmide wurden geerntet und unter Verwendung des Plasmid Preparation Kit von Qiagen aufgereinigt. Die AAV-Bibliothek-Genome wurden in chimäre Wildtyp- und Bibliothek-AAV-Kapside (AAV-Transfer-Shuttle) verpackt, indem 2x10⁸ 293T-Zellen in 10 Zellkulturschalen (15 cm) mit dem Plasmid pVP3cm (enthält das Wildtyp-Cap-Gen mit modifizierter Kodonnutzung ohne die terminalen inverted repeats) [27], dem Bibliothek-Plasmiden und dem pXX6-Helferplasmid [28] transfiziert wurden, wobei das Verhältnis zwischen den Plasmiden 1:1:2 betrug. Die resultierenden AAV-Bibliothek-Transfer-Shuttle wurden dazu verwendet, 2x10⁸ 293T-Zellen in Zellkulturschalen (15 cm) bei einer MOI von 0,5 replikativen Einheiten pro Zelle zu infizieren. Die Zellen wurden mit Ad5 (bereitgestellt durch das Laboratoire des Therapie Genique, Frankreich) bei einer MOI von 5 Plaquebildenden Einheiten (pfu/Zelle) superinfiziert. Die finale AAV-Display-Bibliothek wurde nach 48 Stunden aus den Überständen geerntet. Die Überstände wurden unter Verwendung von VivaSpin-Säulen (Viva Science, Hannover, Deutschland) aufkonzentriert und durch Iodixanol-Dichtegradienten-Ultrazentrifugation wie zuvor beschrieben [29] aufgereinigt und durch real-time-PCR unter Verwendung der cap-spezifischen Primer 5' - GCAGTATGGTTCTGTATCTACCAACC - 3' (SEQ ID NO:14) und 5' - GCCTGGAAGAACGCCTTGTGTG - 3' (SEQ ID NO:15) mit dem LightCycler-System (Roche Diagnostics, Mannheim, Deutschland) titriert.

Für die *in vivo* Selektion wurden 1x10¹¹ Partikel der genomischen Bibliothek in die Schwanzvene von FVB/N-Mäusen injiziert. Den Partikeln wurde 8 Tage Zeit für die Verteilung und die Infektion der Zielzellen gegeben. Nach 8 Tagen wurden die Mäuse getötet und die Gehirne wurden entfernt. Die Gesamt-DNA des Gewebes wurde unter Verwendung des DNeasy Tissue Kits (Qiagen) extrahiert. Die Zufallsoligonukleotide, die in AAV-Partikeln der Bibliothek enthalten waren und sich in dem Gewebe von Interesse angereichert hatten, wurden durch Nested-PCR unter Verwendung der Primer 5'-ATGGCAAGCCACAAGGACGATG - 3' (SEQ ID NO:16) und 5' - CGTGGAGTACTGTGTGATGAAG - 3' (SEQ ID NO:17) für die erste PCR und den Primern 5' - GGTTCTCATCTTTGGGAAGCAAG - 3' (SEQ ID NO:18) und 5'-TGATGAGAATCTGTGGAGGAG - 3' (SEQ ID NO:19) für die zweite PCR amplifiziert. Die mittels PCR amplifizierten Oligonukleotide wurden verwendet, um sekundäre Bibliothek für drei weitere Selektionsrunden herzustellen. Die sekundären Bibliotheken wurden wie die primären Bibliotheken erzeugt (siehe oben), jedoch ohne den zusätzlichen Schritt der Herstellung von Transfer-Shutteln. Die sekundäre Plasmid-Bibliothek wurde verwendet, um 2x10⁸ 293T-Zellen in Zellkulturschalen (15 cm) bei einem Verhältnis von 25 Bibliothek-Plasmiden pro Zelle zu transfizieren, wobei das Transfektionsmittel Polyfect (Qiagen) verwendet wurde. Nach jeder Selektionsrunde wurden einige Klone sequenziert. Das angewendete Selektionsverfahren ist in Fig. 1 abgebildet. Ergebnisse: Nach fünf Selektionsrunden wurden mehrere Gehirnbindenden Kapside sequenziert. Kapside, welche die Peptidsequenz NRGTEWD (SEQ ID NO:1) umfassten, banden besonders stark an Zellen und Gewebe des Gehirns und Rückenmarks (siehe unten). Eine weitere Gruppe von Peptiden, die ebenfalls eine Spezifität für Gehirn und Rückenmark zeigten, umfasste die Peptide ADGVQWT (SEQ ID NO:2), DDGVSWK (SEQ ID NO:3), SDGLTWS (SEQ ID NO:4) und SDGLAWV (SEQ ID NO:5). Diese Peptide enthielten das allgemeine Motiv XDGXXWX (SEQ ID NO:6). Die in den verschiedenen Selektionsrunden gewonnenen Peptide sind in Fig. 2 gezeigt.

### Beispiel 2: Herstellung und Quantifizierung rekombinanter AAV-Vektoren in HEK293T-Zellen

Die in Beispiel 1 angereicherten Klone wurden als rekombinante AAV-Vektoren hergestellt und auf ihr Transduktionsprofil untersucht. Rekombinante AAV-Vektoren wurden durch dreifache Transfektion von HEK293T-Zellen erzeugt. Die Zellen wurden bei 37°C, 5% CO₂ in Dulbeccos modifiziertem Eagle Medium (Invitrogen, Carlsbad, USA), das mit 1% Penicillin/Streptomycin und 10% fetalem Kälberserum supplementiert war, angezüchtet. Plasmid-DNA wurde mit dem Transfektionsmittel Polyfect (Qiagen, Hilden, Deutschland) in 293T-Zellen transfiziert. Vier Tage nach der Transfektion wurden die Zellen geerntet, lysiert und die Vektoren wurden mittels Iodixanol-Dichtegradienten-Ultrazentrifugation aufgereinigt, wie es zuvor beschrieben wurde [29]. Für die Transfektionen wurde pXX6 als adenovirales HelferPlasmid [28], das Luciferase-Gen pUF2-CMV-luc [27] oder das GFP-Gen pTR-CMV-GFP [30], sowie ein Plasmid, das für das AAV-Kapsid von Interesse kodiert, verwendet. Plasmide, die für die AAV-Kapsid-Mutanten kodierten, welche zuvor aus der AAV-Bibliothek selektiert worden waren, und Wildtyp-Kontrollen waren modifiziertes pXX2-187 [31] bzw. pXX2 [28]. Die Inserts wurden wie beschrieben zu Bibliothek-Inserts verarbeitet (siehe oben). Für die Quantifizierung der rekombinanten Vektoren wurden die genomischen Titer durch quantitative real-time PCR unter Verwendung der folgenden CMV-spezifischen Primer 5' - GGCGGAGTTGTTACGACAT - 3' (SEQ ID NO:20) und 5' - GGGACTTTCCCTACTTGGCA - 3' (SEQ ID NO:21) mit dem LightCycler-System bestimmt, wie es zuvor beschrieben wurde [32].

Ergebnisse: Es wurde herausgefunden, dass die Ausbeute in Bezug auf die Vektor-Titer bei rekombinanten Vektoren mit Luciferase-Reporter-Gen vergleichbar zu Vektoren war, die ein Wildtyp-AAV2-Kapsid trugen, was darauf schließen ließ, dass die angereicherten Peptide den Zusammenbau des Kapsids oder die Verpackung des Gens nicht negativ beeinflussen.

### Beispiel 3: Untersuchung des Tropismus der rekombinanten AAV-Vektoren in vivo

Um den Tropismus der angereicherten Peptide *in vivo* untersuchen zu können, wurden die Peptide in das Kapsid eines rekombinanten Vektors eingebracht, der ein Luciferase-Reporter-Gen umfasste. Vektoren mit mutierten Kapsiden sowie Kontrollvektoren wurden in Mäuse injiziert. Die AAV-Vektoren wurden intravenös in einer Dosis von 5x10¹⁰ Vektorgenomen (vg)/Maus verabreicht (n=3 Tiere pro injiziertem AAV-Klon). Am Tag 14 wurden die Tiere mit Isofluran betäubt. Die Luciferase-Expression wurde unter Verwendung eines Xenogen IVIS200-Imaging Systems (Caliper Lifescience, Hopkinton, USA) mit der Software Living Image 4.0 (Caliper) nach intraperitonialer Injektion von 200 µl Luciferin-Substrat (150 mg/kg, Xenogen) pro Maus analysiert. Es wurden repräsentative *in vivo* Biolumineszenz-Abbildungen der Expression des Transgens an unterschiedlichen Positionen (ventral, dorsal, lateral) aufgenommen, wenn die Lumineszenz in relativen Lichteinheiten (Photonen/Sek/cm²) die höchste Intensität erreichte.

Anschließend wurden die Tiere getötet, die Organe von Interesse wurden schnell entfernt, und es wurden sofort Abbildungen der Expression des Transgens in einzelnen Organen aufgenommen. Anschließend wurden die Organe in flüssigem Stickstoff eingefroren und bei -80°C gelagert. Um die Luciferase-Expression zu quantifizieren wurden die Organe in Reporter-Lysepuffer (RLB, Promega, Madison, USA) homogenisiert. Die Bestimmung der Luciferase-Reporter-Gen-Aktivität wurde in ein Luminometer (Mithras LB9 40, Berthold Technologies, Bad Wildbad, Deutschland) in 10-Sekunden Intervallen nach Zugabe von 100 µl Luciferase-Assay-Reagenz (LAR, Promega) durchgeführt, wobei zwischen den Messungen jeweils 2 Sekunden Verzögerung bestand. Die Werte wurden in Bezug auf die Gesamtproteinmenge in jeder Probe normalisiert, wobei der Roti Nanoquant-Proteinassay (Roth, Karlsruhe, Deutschland) verwendet wurde.

Ergebnisse: Die *in vivo* Messung der Biolumineszenz nach 14 Tagen ergab, dass das Peptid NRGTEWD für eine Expression des Transgens im Gehirn sorgte (≤10⁴p/sek/cm²/r) (siehe Fig. 3B). Diese Ergebnisse wurden durch die *ex vivo* durchgeführten Kontrollversuche mit explantierten Organen bestätigt. Ein auf Zufallsbasis ausgewählter Kontrollklon der nicht-selektierten Bibliothek (CVGSPCG) führte zu einer schwachen Gen-Expression, die vornehmlich im Herz und an einigen Stellen des Abdomens auftrat, nicht jedoch im Gehirn (nicht gezeigt). Wildtyp-AAV2 verursachte eine schwache Gen-Expression in Herz, Leber und Skelettmuskel, nicht jedoch im Gehirn (siehe Fig. 3A).

Die Untersuchung der Luciferase-Aktivität von Gewebelysaten aus repräsentativen Organen ergab, dass die Vektoren, die das Gehirn-spezifische NRGTEWD-Kapsid aufwiesen, zu einer sehr starken und spezifischen Gen-Expression im Gehirn führten (1,1x10⁷ RLU/mg Protein, siehe Fig. 5A). In anderen Organgeweben zeigte der NRGTEWD-Luciferase-Vektor kaum Expression.

Die Untersuchungen zeigten ferner, dass die durch den NRGTEWD-Vektor vermittelte gehirnspezifische Expression des Transgens auch über einen langen Zeitraum organspezifisch blieb. Nach intravenöser Verabreichung der AAV2-NRGTEWD-Luciferasev-Vektoren wurde die Expression des Transgens über einen Zeitraum von 168 Tagen gemessen. Die im Bereich des Gehirns emittierte Strahlung wurde dabei quantitativ erfasst. Über den gesamten Zeitraum hinweg erfolgte die Expression des Transgens stabil auf hohem Niveau, und sie war auf das Gehirn beschränkt (siehe Fig. 4).

### Beispiel 4: Analyse der Vektor-Verteilung

Um nachzuprüfen, ob die gehirnspezifische Expression des Transgens von intravenös injizierten NRGTEWD-Vektoren auf einem spezifischen Homing basiert, wurde zunächst die Verteilung der Vektoren 14 Tage nach intravenöser Verabreichung von 5x10¹⁰gp/Maus untersucht. Die Quantifizierung der Vektorgenome erfolgte durch real-time PCR. Zunächst wurde zu verschiedenen Zeitpunkten nach intravenöser Verabreichung von 5x10¹⁰ vg/Maus die Gesamt-DNA aus dem betreffenden Organ unter Verwendung eines Gewebehomogenisators (Precellys 24, Peqlab, Erlangen, Deutschland) und des DNeasy Tissue Kits (Qiagen, Hilden, Deutschland) gemäß den Anweisungen des Herstellers extrahiert. Die DNA wurde unter Verwendung eines Spektralphotometers (Nanodrop ND-2000C, Peqlab) quantifiziert. Die Analyse der AAV-Vektor-DNA in den Geweben wurde durch quantitative real-time PCR unter Verwendung der zuvor beschriebenen CMV-spezifischen Primer durchgeführt, wobei 40 ng Template verwendet wurden, die in Bezug auf die Gesamt-DNA normalisiert wurden.

Ergebnisse: Die Quantifizierung der Vektorgenome durch real-time PCR zeigte ein für das Gehirn spezifisches Homing von NRGTEWD. Die Menge an Vektorgenomen, die im Gehirn nachgewiesen werden konnten (1.6x10⁴ ± 7.1x10³ vg/100 ng Gesamt-DNA) war deutlich höher als die Menge an Vektorgenomen, die in einem anderen Organ nachgewiesen wurde (Fig. 5B). Um die direkte Korrelation zwischen Vektor-Homing und Expression des Transgens zu ermitteln, wurde die Vektorverteilung von Wildtyp-AAV2, des Kontrollpeptids CVGSPCG und des gehirnspezifischen Peptids NRGTEWD 14 Tage nach intravenöser Verabreichung von 5x 10¹⁰ gp/Maus gemessen, d.h. zu dem Zeitpunkt, an dem die Expression des Transgens bestimmt wurde (siehe oben). Die von Wildtyp-AAV2-Vektoren bereitgestellten Genome konnten hauptsächlich aus Leber und Milz gewonnen werden, wobei auch die Genome von Vektoren, die das Kontrollpeptid aufwiesen, hauptsächlich aus der Milz gewonnen wurden (Daten nicht gezeigt). Insgesamt waren die Menge an Vektor-Genomen, die in der Milz nachgewiesen wurden, bei allen untersuchten KapsidVarianten verhältnismäßig ähnlich, was auf einen unspezifischen Aufnahmemechanismus für die Partikel im retikulo-endothelialen System verweist, der unabhängig von der Bereitstellung und der Expression des Transgens ist. Im Gegensatz dazu ähneln die Verteilungsdaten von Genomen, die durch Vektoren bereitgestellt wurden, welche das Peptid NRGTEWD aufwiesen, den Expressionsdaten des Transgens, wobei eine hochspezifische Anreicherung im Gehirn beobachtet werden kann. Die Menge von im Gehirn nachgewiesenen Vektoren, die das Peptid NRGTEWD zeigten, war etwa 168-fach höher als in Gehirnen, denen ein Wildtyp-Vektor oder ein Kontroll-Kapsid-Vektor injiziert wurde. Insgesamt zeigen diese Daten, dass eine gehirnspezifische Expression des Transgens, die durch NRGTEWD-Vektoren vermittelt wird, durch ein Gewebespezifisches Homing von zirkulierenden Partikeln erreicht wird.

### Beispiel 5: Immunhistochemie und Histologie

Es wurde eine Immunhistochemie durchgeführt, um 14 Tage nach intravenöser Verabreichung des rAAV-GFP-Vektors, der das Peptid NRGTEWD aufweist, bzw. des Wildtyp-AAV-Kapsids als Kontrolle die Expression des Transgens auf zellulärer Ebene im Gehirn sowie in einem Kontroll-Organ sichtbar zu machen. Die Gehirne der Tiere wurden mit 4% (w/v) Paraformaldehyd fixiert. Die Gewebe wurden in Paraffin eingebettet. Schnitte einer Stärke von 2 µm wurden von Wachs entfernt, rehydratisiert und für die Immunhistochemie verwendet. Die Immunhistochemie wurde mit polyklonalen Antikörpern gegen GFP (A-11122, Invitrogen) oder CD31 (AB28364, Abcam, Cambridge, USA) durchgeführt. Die Aktivität der endogenen Peroxidase wurde mit 1% H₂O₂ in Methanol für 30 Minuten inaktiviert. Vor der Färbung mit CD31 wurden die Schnitte in Citratpuffer (pH 6,0) für 20 Minuten auf 100°C erhitzt. Nach Waschen in PBS wurden die Schnitte für 30 Minuten mit PBS, 10% Ziegenserum (Vector Lab, Burlingame, USA) und 2% Milchpulver (Roth) inkubiert. Primären Antikörpern wurde es erlaubt, für 1 Stunde bei 37°C zu binden. Nach Waschen in PBS wurden die Schnitte für 30 Minuten mit einem sekundären, biotinylierten Ziege-Anti-Kaninchen-Antikörper (Vector Lab) inkubiert. Gebundene Antikörper wurden unter Verwendung des VECTASTAIN-Elite-ABC-Kits (Vector Lab) und 3,3'-Diaminobenzidin (DAB, Sigma-Aldrich, St. Louis, USA) visualisiert. Ausgewählte Schnitte wurden mit Hemalum gegengefärbt.

Ergebnisse: In den Gehirnen von Mäusen, denen rAAV-NRGTEWD injiziert wurde, ergab eine mikroskopische Untersuchung eine intensive Färbung der Endothelzellen über die gesamte Mikrovaskulatur hinweg und in einem geringfügig geringeren Ausmaß in den größeren Gefäßen (Daten nicht gezeigt). Im Gegensatz dazu zeigte Gehirn-Gewebe von Mäusen, denen Wildtyp-AAV2-Vektor injiziert wurde, keine Färbung. Um die Gewebespezifität zu bestätigen, wurde die Leber als Kontrollorgan analysiert (ein Gewebe, von dem bekannt ist, dass es häufig eine hohe Expression eines Transgens nach Injektion von Wildtyp-AAV2-Vektor zeigt). In der Leber wurde eine Färbung von Hepatozyten nach der Verabreichung von Wildtyp-RAAV2-Vektor beobachtet, jedoch keine Färbung nach Verabreichung des rAAV2-NRGTEWD-Vektors. Die endotheliale Abstammung der mit den Vektoren transduzierten Zellen wurde durch CD31-Färbung bestätigt, wobei das durch die GFP-Färbung erhaltene Muster in seriellen Schnitten der Gehirne von Mäusen, denen rAAV2-NRGTEWD injiziert wurde, bestätigt wurde (Daten nicht gezeigt).

Die Untersuchung des Rückenmarks von Mäusen, denen rAAV-NRGTEWD injiziert wurde, ergab ebenfalls eine intensive Färbung der Endothelzellen der Mikrovaskulatur, was zeigt, dass nicht nur die Endothelzellen des Gehirns, sondern vielmehr die Endothelzellen des gesamten Zentralnervensystems mit den erfindungsgemäßen Peptiden transduzierbar sind (Daten nicht gezeigt).

### Beispiel 6: Herstellung und Quantifizierung rekombinanter AAV-Vektoren mit Hilfe des Baculovirus Expressionssystem in Sf9 Insektenzellen

Zur Produktion rekombinanter AAV-Vektoren in Sf9-Insektenzellen [33-35] wurde das modifizierte AAV2 Genom mit dem für die Peptidinsertion kodierenden Oligonukleotid-Insert im cap-Gen (siehe oben) in das Donorplasmid pFASTBAC Dual (Life Technolgies, Darmstadt, Deutschland) kloniert. In das Donorplasmid wurde ferner ein artifizielles Intron inseriert, das den polh-Promoter umfasste, wodurch das Plasmid pFBD-Repᵢₙ/Capᵢₙ entstand [35]. Zur Erstellung des Donorplasmids pFB-CAG-eGFP wurden der CAG-Promoter und das eGFP-Gen zusammen mit dem SV40-Polyadenylierungssignal und den AAV2-ITRs in das Plasmid pFASTBAC1 (Life Technologies) kloniert. Die Donorplasmide wurden zur Transformation von DH10Bac *E. coli* Zellen verwendet, aus denen anschließend rekombinante Bacmide isoliert wurden, welche das rekombinante AAV-Genom bzw. die eGFP-Transgencassette enthielten. Die Bacmide (9µg) wurden zur Transfektion von jeweils 1x10⁶ Sf9 Zellen im 6-Well Format mittels FectoflyTransfektionsreagenz (Polyplus Transfection/VWR International GmbH, Darmstadt, Deutschland) verwendet. Nach dreitägiger Inkubation der transfizierten Sf9-Insektenzellen bei 27°C in Insect X-Press Medium (Lonza, Köln, Deutschland) mit 1% Gentamycin (Lonza) wurden 500µl der im Zellkulturüberstand befindlichen rekombinanten Baculoviren zur Amplifikation mit frischen 2,5x10⁷ Sf9 Zellen in T175 Zellkulturflaschen für weitere drei Tage bei 27°C in Insect X-Press Medium (Lonza)mit 1% Gentamycin inkubiert. Die auf diese Weise amplifizierten Baculoviren wurden zur Infektion von frischen Sf9 Zellen verwendet, um rekombinante AAV-Vektoren herzustellen. Zu diesem Zweck wurden rekombinantes Baculovirus mit inseriertem AAV-Genom und rekombinantes Baculovirus mit inserierter eGFP-Transgencassette vermischt und zusammen in 400 ml Insect-XPress Medium mit 1% Gentamycin im 1l Erlenmeyerkolben zur Infektion von 6x10⁸ Insektenzellen verwendet. Die Zellen wurden anschließend bei 27°C unter Schütteln (110 UpM) inkubiert. Vier Tage nach der Infektion wurden die Zellen geerntet, lysiert und die AAV-Vektoren wurden mittels Iodixanol-Dichtegradienten-Ultrazentrifugation aufgereinigt, wie es zuvor beschrieben wurde [29]. Für die Quantifizierung der rekombinanten Vektoren wurden die genomischen Titer durch quantitative real-time PCR unter Verwendung der CMV-spezifischen Primer von SEQ ID NO:20 und SEQ ID NO:21 mit dem LightCycler-System bestimmt, wie es zuvor beschrieben wurde [32].

Ergebnisse: Durch Verwendung des Baculovirus-Expressionsystems konnten in Sf9 Insektenzellen höhere Titer rekombinanter AAV-Vektoren erzielt werden als bei der Produktion in HEK293T-Zellen nach Dreifach-Transfektion. Während die Ausbeute der Virusproduktion in HEK293T-Zellen bei maximal 1,9x10⁴ genomischen Partikeln pro Zelle lag, wurde bei Sf9-Insektenzellen eine Ausbeute von bis zu 7,9x10⁴ genomischen Partikeln pro Zelle beobachtet. Ferner konnte beobachtet werden, dass rAAV2-NRGTEWD-Vektoren, die in Sf9-Insektenzellen hergestellt wurden, eine erhöhte Affinität zu neuronalen Zellen aufwiesen als entsprechende rekombinante Vektoren, die in HEK293T-Zellen erzeugt wurden (Daten nicht gezeigt). Somit bietet die Wahl des konkreten Herstellungsverfahrens für die rekombinanten Vektoren die Möglichkeit, die Spezifität der Vektoren für die Neuronen bzw. Endothelzellen weiter zu erhöhen.

### LITERATURLISTE

[1] Tenenbaum et al., 2003, Curr Gene Ther, 3:545-565
[2] Work et al., 2006, Mol Ther, 4:683-693
[3] Shi et al., 2006, Hum Gene Ther, 17:353-361
[4] US 2007/0172460 A1
[5] Michelfelder et al., 2009, PLOS One, 4(4):e5122
[6] Kaplitt et al., 2007, Lancet, 369(9579): p. 2097-105
[7] Gray et al., 2010, Mol Ther, 18(3): p. 570-8
[8] Shevtsova et al., 2005, Exp Physiol. 90(1): p. 53-9
[9] Gray et al., 2011, Hum Gene Ther, 22(9): p. 1143-53
[10] Aebischer et al., 1996, Nat Med, 2(6): p. 696-9
[11] McCown et al., 1996, Brain Res, 713(1-2): p. 99-107
[12] During et al., 1998, Gene Ther. 5(6): p. 820-7
[13] Nagabhushan Kalburgi et al., 2013, Discov Med, 15(81): p. 111-9
[14] Alonso et al., 2013, Mol Ther Nucleic Acids, 2: p. e73
[15] Shi and Bartlett, 2003, Mol Ther, 7:515-525
[16] Loiler et al., 2003, Gene Ther, 10:1551-1558
[17] Rabinowitz et al., 1999, Virology, 265:274-285
[18] Wu et al., 2000, J Virol, 74:8635-8647
[19] Shi et al., 2001, Hum Gene Ther, 12:1697-1711
[20] Warrington et al., 2004, J Virol, 78:6595-6609
[21] Girod et al., 1999, Nat Med, 5:1052-1056
[22] Grifman et al., 2001, Mol Ther, 3:964-975
[23] Opie et al., 2003, J Virol, 77:6995-7006
[24] Kern et al., 2003, J Virol, 77:11072-11081
[25] Russell et al., 1998, J Virol, 72:309-319)
[26] Müller et al., 2003, Nat Biotechnol 21, 1040 - 1046
[27] Waterkamp, et al., 2006, J Gene Med 8, 1307-1319
[28] Xiao et al., 1998, Journal of Virology 72, 2224-2232
[29] Zolotukhin, et al., 1999, Gene Ther 6, 973-985
[30] McCarty et al., 2001, Gene Ther 8, 1248-1254
[31] Michelfelder, et al., 2007, Exp Hematol 35, 1766-1776
[32] Rohr et al., 2005, J Virol Methods 127, 40 - 45
[33] Urabe et al., 2002, Hum Gene Ther 13, 1935-1943
[34] Kohlbrenner et al., 2005, Mol Ther 12, 1217-1225
[35] Chen, 2008, Mol Ther 16, 924-930 (2008)

Ausführungsformen der vorliegenden Erfindung sind:
1. Peptid, Polypeptid oder Protein, das spezifisch an Zellen des Gehirns und/oder des Rückenmarks bindet, dadurch gekennzeichnet, dass es folgendes umfasst:
   (a) die Aminosäuresequenz von SEQ ID NO:1, oder
   (b) eine Aminosäuresequenz, die sich von der Aminosäuresequenz von SEQ ID NO:1 durch Modifikation von einer Aminosäure unterscheidet.
2. Peptid, Polypeptid oder Protein, das spezifisch an Zellen des Gehirns und/oder des Rückenmarks bindet, dadurch gekennzeichnet, dass es die Aminosäuresequenz von SEQ ID NO:6 umfasst.
3. Peptid, Polypeptid oder Protein nach Ausführungsform 2, das folgendes umfasst:
   (a) eine der Aminosäuresequenzen von SEQ ID NO:2-5, oder
   (b) eine Aminosäuresequenz, die sich von einer der Aminosäuresequenzen von SEQ ID NO:1 durch Modifikation von einer Aminosäure unterscheidet.
4. Protein nach einer der Ausführungsformen 1-3, wobei es sich um ein Kapsid-Protein eines viralen Vektors handelt, vorzugsweise um ein Kapsid-Protein eines Adeno-assoziierten Virus (AAV) handelt.
5. Protein nach Ausführungsform 4, wobei es sich um ein Kapsid-Protein eines AAV von einem Serotyp ausgewählt aus der Gruppe bestehend aus den 2, 4, 6, 8 und 9 handelt.
6. Protein nach Ausführungsform 5, wobei es sich um ein Kapsid-Protein eines AAV vom Serotyp 2 handelt.
7. Protein nach Ausführungsform 6, wobei es sich um das VP1-Protein eines AAV vom Serotyp 2 handelt.
8. Protein nach einer der Ausführungsformen 1-7, wobei das Peptid im Bereich der Aminosäuren 550-600 des Kapsids vorliegt.
9. Protein nach einer der Ausführungsformen 1-8, das folgendes umfasst:
   (a) die Aminosäuresequenz von SEQ ID NO:10;
   (b) eine Aminosäuresequenz, die mindestens 80% Identität zu der Aminosäuresequenz von SEQ ID NO:10 aufweist und darüber hinaus die Sequenz von SEQ ID NO:1 umfasst oder eine Aminosäuresequenz, die sich von der Aminosäuresequenz von SEQ ID NO:1 durch Modifikation von einer Aminosäure unterscheidet; oder
   (c) ein Fragment von einer der in (a) oder (b) definierten Aminosäuresequenzen.
10. Virales Kapsid, das ein Peptid, Polypeptid oder Protein nach einer der Ausführungsformen 1-9 umfasst.
11. Nukleinsäure, die für ein Peptid, Polypeptid oder Protein nach einer der Ausführungsformen 1-9 kodiert.
12. Plasmid, das eine Nukleinsäure nach Ausführungsform 11 umfasst.
13. Rekombinanter viraler Vektor, der ein Kapsid und ein darin verpacktes Transgen umfasst, wobei das Kapsid mindestens ein Kapsid-Protein umfasst, welches ein Peptid, Polypeptid oder Protein nach den Ausführungsformen 1-9 umfasst.
14. Rekombinanter viraler Vektor nach Ausführungsform 13, wobei es sich um einen rekombinanten AAV-Vektor handelt.
15. Rekombinanter AAV-Vektor nach Ausführungsform 14, wobei es sich um einen AAV-Vektor eines Serotyps ausgewählt aus der Gruppe bestehend aus den Serotypen 2, 4, 6, 8 und 9 handelt.
16. Rekombinanter AAV-Vektor nach Ausführungsform 15, wobei es sich um einen AAV-Vektor vom Serotyp 2 handelt.
17. Rekombinanter AAV-Vektor nach einer der Ausführungsformen 13-16, wobei das Transgen für eines der folgenden Proteine kodiert: ein Membran- oder Tight-Junction-Protein, wie z.B. ein Claudin oder Occludin, Neuraminidase, z.B. Neuraminidase 1, Glucuronidase, ein Chemokin-Antagonist, wie z.B. CCL2-7ND, Neurotrophischer Faktor der Gliazellen (GDNF), Neprilysin, Cholesterol 24-Hydroxylase, aromatische L-Aminosäure-Decarboxylase, Tyrosynhydroxylase, GTP-Cyclohydrolase I und Survival of Motor Neuron(SMN)-Protein.
18. Rekombinanter AAV-Vektor nach Ausführungsform 17, wobei das Transgen für eine Neuraminidase kodiert.
19. Rekombinanter AAV-Vektor nach einer der Ausführungsformen 13-18, wobei das Transgen in Form einer ssDNA oder einer dsDNA vorliegt.
20. Rekombinanter AAV-Vektor nach einer der Ausführungsformen 13-19 zur Verwendung in einem Verfahren zur Behandlung einer Funktionsstörung oder einer Erkrankung des Gehirns und/oder Rückenmarks in einem Subjekt.
21. Rekombinanter AAV-Vektor zur Verwendung in einem Verfahren nach Ausführungsform 20, wobei die Erkrankung des Gehirns und/oder Rückenmarks ausgewählt ist aus der Gruppe bestehend aus genetisch verursachten Leukodystrophien, wie Adrenoleukodystrophie, Morbus Canavan, Morbus Krabbe, metachromatische Leukodystrophie, Pelizaeus-Merzbacher-Krankheit und Morbus Alexander; neurodegenerativen Erkrankungen, wie amyotrophe Lateralsklerose, Alzheimer, Parkinson, Chorea Huntington und Morbus Pick; chronisch-entzündlichen Erkrankungen des Zentralnervensystems, wie Multiple Sklerose und Guillain-Barre-Syndrom; und Lysosomen-Speichererkrankungen, wie Ceroid-Lipofuszinose und Morbus Fabry.
22. Rekombinanter AAV-Vektor zur Verwendung in einem Verfahren nach einer der Ausführungsformen 20-21, wobei das Subjekt ein Säugetier, vorzugsweise ein Mensch ist.
23. Rekombinanter AAV-Vektor zur Verwendung in einem Verfahren nach einer der Ausführungsformen 20-22, wobei der Vektor für die intravenöse Verabreichung formuliert ist.
24. Zelle, die ein Peptid, Polypeptid oder Protein nach einer der Ausführungsformen 1-9, ein virales Kapsid nach Ausführungsform 10, eine Nukleotidsequenz nach Ausführungsform 11, ein Plasmid nach Ausführungsform 12, oder einen rekombinanten AAV-Vektor nach einer der Ausführungsformen 13-19 umfasst.
25. Pharmazeutische Zusammensetzung, die ein Peptid, Polypeptid oder Protein nach einer der Ausführungsformen 1-9, ein virales Kapsid nach Ausführungsform 10, eine Nukleotidsequenz nach Ausführungsform 11, ein Plasmid nach Ausführungsform 12, oder einen rekombinanten AAV-Vektor nach einer der Ausführungsformen 13-19 umfasst.
26. Verwendung eines Peptids, Polypeptids oder Proteins nach einer der Ausführungsformen 1-9, eines viralen Kapsids nach Ausführungsform 10, einer Nukleotidsequenz nach Ausführungsform 11, eines Plasmids nach Ausführungsform 12 oder eines rekombinanten AAV-Vektors nach einer der Ausführungsformen 13-19 zur Behandlung einer Funktionsstörung oder einer Erkrankung des Gehirns und/oder Rückenmarks in einem Subjekt.
27. Verwendung nach Ausführungsform 26, wobei die Erkrankung des Gehirns und/oder Rückenmarks ausgewählt ist aus der Gruppe bestehend aus genetisch verursachten Leukodystrophien, wie Adrenoleukodystrophie, Morbus Canavan, Morbus Krabbe, metachromatische Leukodystrophie, Pelizaeus-Merzbacher-Krankheit und Morbus Alexander; neurodegenerativen Erkrankungen, wie amyotrophe Lateralsklerose, Alzheimer, Parkinson, Chorea Huntington und Morbus Pick; chronisch-entzündlichen Erkrankungen des Zentralnervensystems, wie Multiple Sklerose und Guillain-Barre-Syndrom; und Lysosomen-Speichererkrankungen, wie Ceroid-Lipofuszinose und Morbus Fabry.

## Patentansprüche

1. Kapsid-Protein eines Adeno-assoziierten Virus (AAV), **dadurch gekennzeichnet, dass** es folgendes umfasst:
(a) die Aminosäuresequenz von SEQ ID NO:1, oder
(b) eine Aminosäuresequenz, die sich von der Aminosäuresequenz von SEQ ID NO:1 durch Modifikation von einer Aminosäure unterscheidet.

2. Kapsid-Protein nach Anspruch 1, wobei es sich um ein Kapsid-Protein eines AAV von einem Serotyp ausgewählt aus der Gruppe bestehend aus den 2, 4, 6, 8 und 9 handelt.

3. Kapsid-Protein nach Anspruch 2, wobei es sich um das VP1-Protein eines AAV vom Serotyp 2 handelt.

4. Kapsid-Protein nach einem der Ansprüche 1-3, das folgendes umfasst:
(a) die Aminosäuresequenz von SEQ ID NO:10;
(b) eine Aminosäuresequenz, die mindestens 80% Identität zu der Aminosäuresequenz von SEQ ID NO:10 aufweist und darüber hinaus die Sequenz von SEQ ID NO:1 umfasst oder eine Aminosäuresequenz, die sich von der Aminosäuresequenz von SEQ ID NO:1 durch Modifikation von einer Aminosäure unterscheidet; oder
(c) ein Fragment von einer der in (a) oder (b) definierten Aminosäuresequenzen.

5. Virales Kapsid, das ein Kapsid-Protein nach einem der Ansprüche 1-4 umfasst.

6. Nukleinsäure, die für ein Kapsid-Protein nach einem der Ansprüche 1-4 kodiert.

7. Plasmid, das eine Nukleinsäure nach Anspruch 6 umfasst.

8. Rekombinanter viraler Vektor, der ein virales Kapsid nach Anspruch 5 und ein darin verpacktes Transgen umfasst.

9. Rekombinanter viraler Vektor nach Anspruch 8, wobei es sich um einen rekombinanten AAV-Vektor handelt.

10. Rekombinanter viraler Vektor nach Anspruch 9, wobei es sich um einen AAV-Vektor eines Serotyps ausgewählt aus der Gruppe bestehend aus den Serotypen 2, 4, 6, 8 und 9 handelt.

11. Rekombinanter viraler Vektor nach einem der Ansprüche 8-10, wobei das Transgen für eines der folgenden Proteine kodiert: ein Membran- oder Tight-Junction-Protein, wie z.B. ein Claudin oder Occludin, Neuraminidase, z.B. Neuraminidase 1, Glucuronidase, ein Chemokin-Antagonist, wie z.B. CCL2-7ND, Neurotrophischer Faktor der Gliazellen (GDNF), Neprilysin, Cholesterol 24-Hydroxylase, aromatische L-Aminosäure-Decarboxylase, Tyrosynhydroxylase, GTP-Cyclohydrolase I und Survival of Motor Neuron(SMN)-Protein.

12. Rekombinanter viraler Vektor nach einem der Ansprüche 8-11 zur Verwendung in einem Verfahren zur Behandlung einer Erkrankung in einem Subjekt, wobei die Erkrankung ausgewählt ist aus der Gruppe bestehend aus genetisch verursachten Leukodystrophien, wie Adrenoleukodystrophie, Morbus Canavan, Morbus Krabbe, metachromatische Leukodystrophie, Pelizaeus-Merzbacher-Krankheit und Morbus Alexander; neurodegenerativen Erkrankungen, wie amyotrophe Lateralsklerose, Alzheimer, Parkinson, Chorea Huntington und Morbus Pick; chronisch-entzündlichen Erkrankungen des Zentralnervensystems, wie Multiple Sklerose und Guillain-Barre-Syndrom; und Lysosomen-Speichererkrankungen, wie Ceroid-Lipofuszinose und Morbus Fabry.

13. Zelle, die ein Kapsid-Protein nach einem der Ansprüche 1-4, ein virales Kapsid nach Anspruch 5, eine Nukleinsäure nach Anspruch 6, ein Plasmid nach Anspruch 7, oder einen rekombinanten viralen Vektor nach einem der Ansprüche 8-11 umfasst.

14. Pharmazeutische Zusammensetzung, die ein Kapsid-Protein nach einem der Ansprüche 1-4, ein virales Kapsid nach Anspruch 5, eine Nukleinsäure nach Anspruch 6, ein Plasmid nach Anspruch 7, oder einen rekombinanten viralen Vektor nach einem der Ansprüche 8-11 umfasst.

15. Kapsid-Protein nach einem der Ansprüche 1-4, virales Kapsid nach Anspruch 5, Nukleinsäure nach Anspruch 6, Plasmid nach Anspruch 7 oder rekombinanter viraler Vektor nach einem der Ansprüche 8-11 zur Verwendung in einem Verfahren zur Behandlung einer Erkrankung in einem Subjekt, wobei die Erkrankung ausgewählt ist aus der Gruppe bestehend aus genetisch verursachten Leukodystrophien, wie Adrenoleukodystrophie, Morbus Canavan, Morbus Krabbe, metachromatische Leukodystrophie, Pelizaeus-Merzbacher-Krankheit und Morbus Alexander; neurodegenerativen Erkrankungen, wie amyotrophe Lateralsklerose, Alzheimer, Parkinson, Chorea Huntington und Morbus Pick; chronisch-entzündlichen Erkrankungen des Zentralnervensystems, wie Multiple Sklerose und Guillain-Barre-Syndrom; und Lysosomen-Speichererkrankungen, wie Ceroid-Lipofuszinose und Morbus Fabry.
